# EUROPEAN PATENT APPLICATION

(11) **EP 2 184 351 A1**
(43) Date of publication of application: **12.05.2010**
(21) Application number: 08018970.7
(22) Date of filing: 30.10.2008
(51) Int. Cl.: C12N 9/88

(54) **Polynucleotides encoding caryophyllene synthase and uses thereof**

(71) Applicant: Max-Planck-Gesellschaft zur Förderung der Wissenschaften e.V., 80539 München (DE); Université de Neuchâtel, 2000 Neuchatel (CH)
(72) Inventor: Degenhardt, Jörg, 06618 Naumburg (DE); Köllner, Tobias, 07768 Großeutersdorf (DE); Gershenzon, Jonathan, 07745 Jena (DE); Crocoll, Christoph, 07749 Jena (DE); Turlings, Ted, 2000 Neuchâtel (CH); Hiltpold, Ivan, 2000 Neuchâtel (CH)
(74) Representative: Vossius & Partner

(57) **Abstract**

Described are polynucleotides encoding a caryophyllene synthase. Also described are recombinant nucleic acid molecules and vectors comprising said polynucleotide as well as to host cells genetically engineered with said polynucleotides. Further described are caryophyllene synthase polypeptides encoded by said polynucleotides as well as binding molecules specifically recognizing said polypeptide. Additionally, described are transgenic plant cells, plant tissues and plants genetically engineered with the caryophyllene synthase-encoding polynucleotide, corresponding methods for the production of said transgenic plant cells, plant tissues and plants and corresponding uses and methods using said polynucleotide for establishing or enhancing resistance against a herbivore, preferably a root-damaging insect in plants, particularly resistance against the corn rootworm in maize plants.

## Description

The present invention relates to polynucleotides encoding a caryophyllene synthase. The present invention furthermore relates to recombinant nucleic acid molecules and vectors comprising said polynucleotide as well as to host cells genetically engineered with said polynucleotides. The present invention also relates to caryophyllene synthase polypeptides encoded by said polynucleotides as well as to binding molecules specifically recognizing said polypeptide. Furthermore, the present invention relates to transgenic plant cells, plant tissues and plants genetically engineered with the caryophyllene synthase-encoding polynucleotide, to corresponding methods for the production of said transgenic plant cells, plant tissues and plants and to corresponding uses and methods using said polynucleotide for establishing or enhancing resistance against herbivores, preferably root-damaging insects in plants, particularly resistance against the corn rootworm in maize plants.

Environmentally desirable protection of crops against arthropod herbivores can inter alia use biological control, which depends on the use of antagonists or enemies of the pest organisms. To be effective, it is crucial that the biological control agents are able to find their prey efficiently enough to minimize damage to the crop.
It was discovered that when herbivores feed, the plants produce volatiles that are attractive to the natural enemies of the herbivore (Dicke, Proceedings of Semiochemicals and Pest Control - Prospects for New Applications, 1990, Wageningen, the Netherlands, pp. 3091-3118; and Turlings, Science 250 (1990), 1251-1853). Thus, plants indirectly defend themselves by enhancing the effectiveness of the natural enemies of the herbivores. The use of predators and parasitoids for biological control is receiving more and more attention and for many years it has been common practice in a number of crops in glasshouse as well as open fields (Van Lenteren, In: Biological control: Measures of Success, eds. G. Gurr & S. Wratten, 2000, Kluwer Academic Publishers, Dordrecht; Kfir, Annual Rev. Entomol. 47 (2002), 701-731). Nevertheless, breeders and agronomists have so far paid little attention to the optimisation of biological control. This is probably due to the relatively recent discovery of the phenomenon of indirect defence, the more complex relationships involved and the difficulties associated with quantification of the effects.
The beetle *Diabrotica virgifera virgifera* (a species of the corn rootworm) is an agronomically important maize pest in North America that attacks the roots of the plants. It has recently been introduced into Europe and is now quickly spreading within Germany and other European countries. Since the larvae of these beetles live in the soil and damage the roots of the maize plant, it has been very difficult and expensive to control this pest through pesticide applications.
In the work of Rasmann et al. (Nature 434 (2005), 732-737), it was recently demonstrated that some maize varieties are capable to defend themselves against Diabrotica utilizing an indirect defence mechanism. After damage by Diabrotica larvae, the roots of these plants release (*E*)-β-caryophyllene (EβC), a sesquiterpene hydrocarbon, into the soil. This compound attracts nematodes from the surrounding soil that feed on the Diabrotica larvae, eventually killing them. Thereby, the release of caryophyllene leads to an overall benefit to the plants in laboratory experiments. The first field experiments also suggest a reduced Diabrotica damage due to caryophyllene emission.
A similar system is known from the aerial parts of maize plants which, when attacked by caterpillars, release a mixture of odorous compounds that attract parasitic wasps, natural enemies of the herbivores. In a study reported by Degen et al. (Plant Physiol. 135 (2004), 1928-1938), European and American maize inbred lines were examined for their capacity to release these odorous compounds. Thereby, it was found out that European maize varieties are mostly able to produce caryophyllene while almost all American varieties have lost this ability.
Previous attempts to control western corn rootworm (WCR) with nematodes have been largely ineffective or required extraordinary large numbers of nematodes (Jackson, 1996; Jackson et al., 1995; Journey et al., 2000; Nishimatsu et al., 1998; Wright et al., 1993). A possible explanation for these failures is the inability of most North American maize lines to emit EβC (Rasmann et al., 2005; Degen et al., 2004). Recently the caryophyllene synthase gene TPS23 was identified, which is responsible for EβC production in maize (WO 2008/006564). North American maize lines possess a fully functional TPS23, but EβC production is absent due to the lack of TPS23 transcript (Köllner et al., 2008). The absence of EβC dramatically reduces attraction of nematodes, as is evident from a field experiment in which a five-fold higher infection by nematodes was found near a variety producing EβC than near a variety without this signal (Rasmann et al., 2005).
The provision of transgenic maize plants expressing TPS23 and attracting nematodes are disclosed in WO 2008/006564. However, the use of a heterologous caryophyllene synthase for producing caryophyllene in maize plants and thereby improving resistance against Diabrotica pest, particularly in North American varieties, has not yet been described.

Thus, the technical problem underlying the present invention is to provide alternative means and methods for enhancing caryophyllene production in the roots of plants in order to increase the plant's resistance against root-damaging insects.

This technical problem is solved by the provision of the embodiments as characterized in the claims.

Accordingly, the present invention relates to polynucleotides selected from the group consisting of
(a) polynucleotides comprising a nucleotide sequence encoding a polypeptide having the amino acid sequence of SEQ ID NO:2 or 4;
(b) polynucleotides comprising the nucleotide sequence shown in SEQ ID NO:1 or 3;
(c) polynucleotides comprising a nucleotide sequence encoding a fragment of the polypeptide encoded by a polynucleotide of (a) or (b), wherein said nucleotide sequence encodes a protein having caryophyllene synthase activity;
(d) polynucleotides comprising a nucleotide sequence the complementary strand of which hybridizes to the polynucleotide of any one of (a) to (c), wherein said nucleotide sequence encodes a protein having caryophyllene synthase activity;
(e) polynucleotides encoding a polypeptide having an amino acid sequence which is at least 50% identical to the amino acid sequence of a polypeptide encoded by a polynucleotide of any one of (a) to (c) and having caryophyllene synthase activity; and
(f) polynucleotides comprising a nucleotide sequence that deviates from the nucleotide sequence defined in (d) or (e) by the degeneracy of the genetic code.

The present invention is based on the isolation of the gene *Ov*TPS6 from the lines d06-01 and f02-04 of *Origanum vulgare* which encode a polypeptide having caryophyllene synthase activity. The polypeptides encoded by these gene sequences have been shown to be able to synthesize (E)-β-caryophyllene (Example 1).
The gene *Ov*TPS6, under the control of a maize ubiquitin promoter, was used for the transformation of the maize variety Hill which is deficient in expressing caryophyllene synthase. The transformation resulted in plants that produced EβC constitutively (Example 3).
Field experiments were carried out to compare maize plants that were transformed with the EβC synthase gene *Ov*TPS6 with control lines. It was shown that the transgenic plants received significantly less root damage than the controls. In accordance, adult WCR emergence per plant was reduced to less than half with the transformed plants as compared to the control plants (Example 4)
Thus, it is contemplated that the polynucleotide of the present invention may be used for providing plants that have an increased resistance against root-damaging insects, either by producing corresponding transgenic plants or by applying molecular marker-assisted non-transgenic breeding methods.
In connection with the present invention, the term "resistant" or "resistance" refers to the property of a given plant or plant species to protect itself against an attack by a certain root-damaging insect, whereby said protection may range from a significant reduction to a complete inhibition of herbivory. The type of resistance envisaged in connection with the present invention is based on a so-called tritrophic interaction between a plant, a root-damaging insect and an entomopathogenic nematode (cf. Bouwmeester, Proceedings of BCPC International Congress Crop Science & Technology 2003, 10-12 November 2003, Glasgow, Scotland, Vol 2, 1123-1134). In such a system, the feeding of a herbivore (e.g. a root-damaging insect) leads to the release of one or more volatile compounds (e.g. caryophyllene) which attract one or more predators specialized for the herbivore (e.g. entomopathogenic nematodes). This is described in Rasmann (loc. cit.) for a tritrophic interaction between maize plants, the corn rootworm and the nematode *Heterorhabditis megidis.* As is apparent from the tritrophic plant defense system on which the present invention is based, the resistance conferred to a plant by expressing the polynucleotide of the invention presupposes that at least one suitable entomopathogenic nematode that is attracted by caryophyllene and that attacks the root-damaging insect is available in the soil surrounding the plant. In order to fulfill this requirement, suitable nematodes may be naturally present in the soil and/or may be added as a biological pest control agent. The nematodes may be added to the soil together with a nutrient suitable for them. As is typical for biological pest control systems, the use of a biological enemy of the pest organism might not lead to the complete elimination of the pest, but will reduce the levels of the pest organism below those resulting in agronomic significant damage. The resulting levels of the pest organism will be dependent on the populations of entomopathogenic nematodes that are either naturally occurring in the soil or will be added to the soil. The already established agronomic procedures of insecticide treatment to reduce the concentration of the adult Diabrotica beetle, soil insecticide treatments to reduce the concentration of the Diabrotica larvae and insecticide treatment of the seeds can be combined with the herein described biological defense strategies.
Successfully established resistance in a plant according to the present invention may be measured by assays described in the prior art, in particular in Rasmann (loc. cit.). Accordingly, a plant produced according to the present invention may be exposed to a root-damaging insect and, subsequently, its capacity to attract entomopathogenic nematodes as compared to a corresponding control plant may be examined. This may be done by using a six-arm olfactometer as disclosed in Rasmann, (loc. cit.) or described in Example 6. In such an assay, a plant according to the present invention shows an attraction of entomopathogenic nematodes which is significantly higher than in the control plant, preferably at least 20%, more preferably at least 50% and most preferably at least 100% higher.
Alternatively, resistance can be detected by measuring the number of herbivores (larvae) around one plant, the number of nematode-infected herbivores around one plant or the number of adult insects emerging around one plant, in comparison to a corresponding control plant. Corresponding assays which were carried out in the field are also described in Rasmann (loc. cit.) or described in Example 4. Accordingly, with respect to plants of the present invention, the number of herbivores (larvae) around one plant is significantly reduced, preferably by at least 10%, more preferably by at least 20%, as compared to a control plant. Furthermore, the amount of nematode-infected herbivores (larvae) is increased in plants according to the present invention significantly, preferably by at least 100%, more preferably by at least 200% and even more preferably by at least 300% as compared to the control plant. The number of adult insects emerging around one plant is significantly decreased for plants of the present invention as compared to the control plant, preferably by at least 10%, more preferably by at least 20% and even more preferably by at least 30%.
In the context of the present invention, the term "herbivore" relates to insects, in particular insect larvae, that feed on plants.
The term "root-damaging insect" as used herein refers to insect larvae which feed on plant roots. In the framework of the present invention, this term particularly refers to larvae of the genus Diabrotica (interchangeably also referred to herein as "corn rootworm") and, especially, to larvae of the species *Diabrotica virgifera,* and more preferably to larvae of the subspecies *Diabrotica virgifera virgifera* (Western corn rootworm, WCR).
The term "caryophyllene synthase activity" in connection with the present invention refers to the activity of a polypeptide encoded by the polynucleotide of the invention to catalyze the conversion of (*E*,*E*)-farnesyl diphosphate (FPP) to (*E*)-β-caryophyllene (EβC).
The activity can be tested for by assays for caryophyllene synthase activity known in the prior art, such as in Cai, Phytochemistry 61 (2002), 523-529 or Köllner et al., 2008. Preferably, the caryophyllene synthase of the invention does not accept geranyl diphosphate (GPP) or geranyl-geranyl diphosphate (GGPP) as substrate. Furthermore, the caryophyllene synthase of the invention preferably produces α-humulene as minor side product.

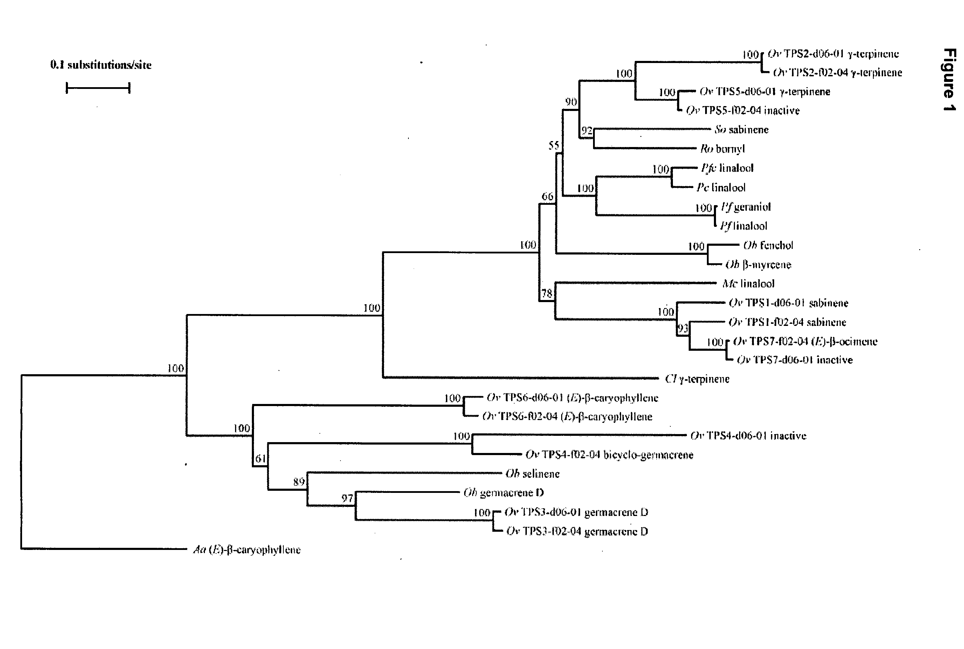

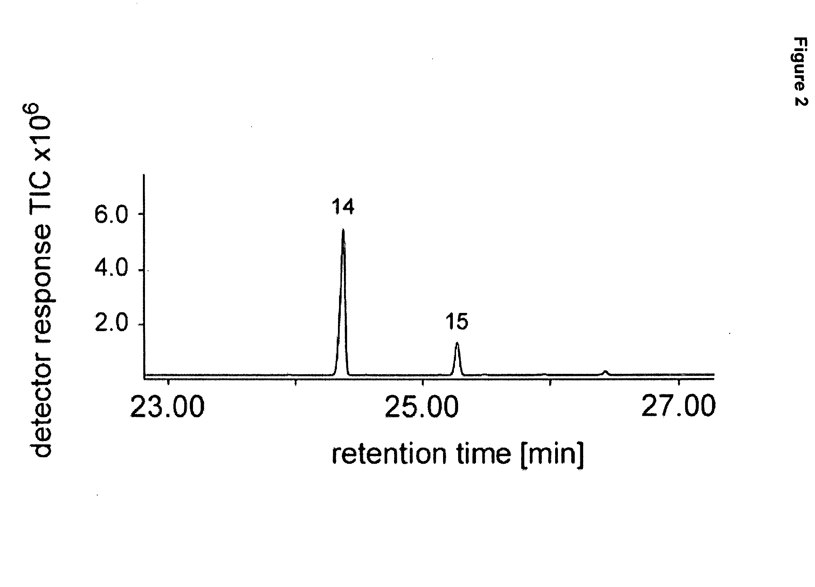

The polynucleotides of the invention preferably comprise the 1.665 bp coding sequence shown in SEQ ID NO: 1 and the 1.665 bp coding sequence shown in SEQ ID NO: 3, particularly preferably, encoding a protein with a predicted molecular mass of 64.7 kDa or 64.6 kDa. As is evident from Figure 1, *Ov*TPS6 shows relatively low sequence identity to other (*E*)-β-caryophyllene synthases, e.g. with that from *Artemisia annua* (Cai, Phytochemistry 61 (2002), 523-529) an identity of only 41.2% on the amino acid level. From Lamiaceae species other than *Origanum vulgare,* no caryophyllene synthase coding sequence has yet been identified (see Figure 1). The (*E*)-β-caryophyllene synthases from *Origanum vulgare* and maize (TPS23) show a sequence identity of only 34%. Numerous amino acids throughout the coding sequence are highly conserved among members of the terpene synthase (TPS) family. The most characteristic element is an Asp-rich DDxxD motif present in the C-terminal part of the protein TPS6 that is involved in the binding of the divalent metal cofactor (Starks et al., 1997). Furthermore, *Ov*TPS6 does not display a signal peptide.
It is not possible to predict, solely based on the amino acid sequence of a terpene synthase, which exact reaction the enzyme catalyzes. It is even so that the assignment to the family of terpene synthases may be problematic since, for example, prenyl synthases are also known to comprise the DDxxD sequence motif (Gershenzon and Kreis, 1999). Thus, the activity of *Ov*TPS6 could only be determined by recombinant expression of the enzyme and assay of the activity in a biochemical test system. Expression of *Ov*TPS6 resulted in an enzyme producing (*E*)-β-caryophyllene and α-humulene from FPP (see Figure 2).

As indicated above, in addition to polynucleotides comprising a nucleotide sequence which encodes the amino acid sequence of SEQ ID NO: 2 or 4 or comprising the nucleotide sequence of SEQ ID NO: 1 or 3, the present invention also refers to fragments of these polynucleotides as well as to polynucleotides comprising a nucleotide sequence the complementary strand of which hybridizes to these polynucleotides, wherein said fragments and hybridizing polynucleotides encode a protein having caryophyllene synthase activity.
The present invention also relates to polynucleotides which encode a polypeptide, which has a homology, that is to say a sequence identity, of at least 30%, preferably of at least 40%, more preferably of at least 50%, even more preferably of at least 60% and particularly preferred of at least 70%, especially preferred of at least 80% and even more preferred of at least 90% to the entire amino acid sequence as indicated in SEQ ID NO: 2 or 4, the polypeptide having caryophyllene synthase activity.
Moreover, the present invention relates to polynucleotides which encode a polypeptide having caryophyllene synthase activity and the nucleotide sequence of which has a homology, that is to say a sequence identity, of at least 40%, preferably of at least 50%, more preferably of at least 60%, even more preferably of more than 65%, in particular of at least 70%, especially preferred of at least 80%, in particular of at least 90% and even more preferred of at least 95% when compared to the coding sequence shown in SEQ ID NO: 1 or 3.
It is particularly preferred that polynucleotides of the invention that encode a polypeptide having caryophyllene synthase activity show the structural characteristics described above for *Ov*TPS6.
The present invention also relates to polynucleotides, which encode a polypeptide having caryophyllene synthase activity and the sequence of which deviates from the nucleotide sequences of the above-described polynucleotides due to the degeneracy of the genetic code.
The invention also relates to polynucleotides comprising a nucleotide sequence which is complementary to the whole or a part of one of the above-mentioned sequences.
The polynucleotide of the invention is understood to be isolated. The term "isolated" means that the polynucleotide addressed herein is not in the form as it occurs naturally, if it indeed has a naturally occurring counterpart. Accordingly, the other compounds of the invention described further below are understood to be isolated.

In the context of the present invention the term "hybridization" means hybridization under conventional hybridization conditions, preferably under stringent conditions, as for instance described in Sambrook and Russell (2001), Molecular Cloning: A Laboratory Manual, CSH Press, Cold Spring Harbor, NY, USA. In an especially preferred embodiment, the term "hybridization" means that hybridization occurs under the following conditions:

| | |
|---|---|
| Hybridization buffer: | 2 × SC; 10 x Denhardt solution (Fikoll 400 + PEG + BSA; ratio 1:1:1); 0.1% SDS; 5 mM EDTA; 50 mM Na₂HPO₄; |
| | 250 µg/ml of herring sperm DNA; 50 µg/ml of tRNA; |
| | or |
| | 0.25 M of sodium phosphate buffer, pH 7.2; |
| | 1 mM EDTA |
| | 7% SDS |
| Hybridization temperature T | = 60°C |
| Washing buffer: | 2 x SSC; 0.1% SDS |
| Washing temperature T | = 60°C. |

Polynucleotides which hybridize with the polynucleotides of the invention can, in principle, encode a polypeptide having caryophyllene synthase activity from any organism expressing such polypeptides or can encode modified versions thereof.
Polynucleotides which hybridize with the polynucleotides disclosed in connection with the invention can for instance be isolated from genomic libraries or cDNA libraries of bacteria, fungi, plants or animals. Preferably, such polynucleotides are from plant origin, particularly preferred from a plant belonging to the dicotyledons, more preferably from the family of Lamiaceae and even more preferably from the genus Origanum. Preferably, the polynucleotide of the invention is a variant, preferably an ortholog of a polynucleotide comprising SEQ ID NO:1 or 3 and may for example comprise a nucleotide sequence that originates from an agronomically important species. Alternatively, such polynucleotides can be prepared by genetic engineering or chemical synthesis.
Such hybridizing polynucleotides may be identified and isolated by using the polynucleotides described hereinabove or parts or reverse complements thereof, for instance by hybridization according to standard methods (see for instance Sambrook and Russell (2001), Molecular Cloning: A Laboratory Manual, CSH Press, Cold Spring Harbor, NY, USA). Polynucleotides comprising the same or substantially the same nucleotide sequence as indicated in SEQ ID NO: 1 or 3 or parts thereof can, for instance, be used as hybridization probes. The fragments used as hybridization probes can also be synthetic fragments which are prepared by usual synthesis techniques, and the sequence of which is substantially identical with that of a polynucleotide according to the invention.
The molecules hybridizing with the polynucleotides of the invention also comprise fragments, derivatives and allelic variants of the above-described polynucleotides encoding a polypeptide having caryophyllene synthase activity. Herein, fragments are understood to mean parts of the polynucleotides which are long enough to encode the described polypeptide, preferably showing the biological activity of a polypeptide of the invention as described above. In this context, the term derivative means that the sequences of these molecules differ from the sequences of the above-described polynucleotides in one or more positions and show a high degree of homology to these sequences, preferably within the preferred ranges of homology mentioned above.
Preferably, the degree of homology is determined by comparing the respective sequence with the nucleotide sequence of the coding region of SEQ ID NO: 1 or 3. When the sequences which are compared do not have the same length, the degree of homology preferably either refers to the percentage of nucleotide residues in the shorter sequence which are identical to nucleotide residues in the longer sequence or to the percentage of nucleotides in the longer sequence which are identical to nucleotide sequence in the shorter sequence. The degree of homology can be determined conventionally using known computer programs such as ClustalX (Thompson et al., 1997) or the DNASTAR program with the ClustalW analysis. This program can be obtained from DNASTAR, Inc., 1228 South Park Street, Madison, Wl 53715 or from DNASTAR, Ltd., Abacus House, West Ealing, London W13 0AS UK (support@dnastar.com) and is accessible at the server of the EMBL outstation.
When using the Clustal analysis method to determine whether a particular sequence is, for instance, 80% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.
Preferably, the degree of homology of the hybridizing polynucleotide is calculated over the complete length of its coding sequence. It is furthermore preferred that such a hybridizing polynucleotide, and in particular the coding sequence comprised therein, has a length of at least 300 nucleotides, preferably at least 500 nucleotides, more preferably of at least 750 nucleotides, even more preferably of at least 1000 nucleotides, and most preferred of at least 1500 nucleotides.
Preferably, sequences hybridizing to a polynucleotide according to the invention comprise a region of homology of at least 90%, preferably of at least 93%, more preferably of at least 95%, still more preferably of at least 98% and particularly preferred of at least 99% identity to an above-described polynucleotide, wherein this region of homology has a length of at least 500 nucleotides, more preferably of at least 750 nucleotides, even more preferably of at least 1000 nucleotides and most preferred of at least 1500 nucleotides.

Homology, moreover, means that there is a functional and/or structural equivalence between the corresponding polynucleotides or polypeptides encoded thereby. Polynucleotides which are homologous to the above-described molecules and represent derivatives of these molecules are normally variations of these molecules which represent modifications having the same biological function. They may be either naturally occurring variations, for instance sequences from other ecotypes, varieties, species, etc., or mutations, and said mutations may have formed naturally or may have been produced by deliberate mutagenesis. Furthermore, the variations may be synthetically produced sequences. The allelic variants may be naturally occurring variants or synthetically produced variants or variants produced by recombinant DNA techniques. Deviations from the above-described polynucleotides may have been produced, e.g., by deletion, substitution, insertion and/or recombination.
The polypeptides encoded by the different variants of the polynucleotides of the invention possess certain characteristics they have in common. These include for instance biological activity, molecular weight, immunological reactivity, conformation, etc., and physical properties, such as for instance the migration behavior in gel electrophoreses, chromatographic behavior, sedimentation coefficients, solubility, spectroscopic properties, stability, pH optimum, temperature optimum etc.
The biological activity of a polypeptide of the invention, in particular the capacity to synthesize caryophyllene, can be tested in conventional enzyme assays using the substrate of the polypeptide or a suitable modified form thereof.
The polynucleotides of the invention can be DNA molecules, in particular genomic DNA or cDNA. Moreover, the polynucleotides of the invention may be RNA molecules. The polynucleotides of the invention can be obtained for instance from natural sources or may be produced synthetically or by recombinant techniques, such as PCR, and include modified or derivatized nucleic acid molecules as can be obtained by applying techniques described in the pertinent literature.

In a further embodiment, the invention relates to polynucleotides (including oligonucleotides) specifically hybridizing with a polynucleotide of the invention as described above or with the complementary strand thereof. Such polynucleotides have a length of preferably at least 10, in particular at least 15, and particularly preferably of at least 50 nucleotides. Advantageously, their length does not exceed a length of 1000, preferably 500, more preferably 200, still more preferably 100 and most preferably 50 nucleotides. They are **characterized in that** they specifically hybridize to the polynucleotides of the invention, that is to say that they do not or only to a very minor extent hybridize to nucleotide sequences encoding another terpene synthase or another caryophyllene synthase such as those from dicotyledons described in the prior art.
The polynucleotides of the present embodiment can be used for instance as primers for amplification techniques such as the PCR reaction or as a hybridization probe to isolate related genes. The hybridization conditions and homology values described above in connection with the polynucleotide encoding a polypeptide having caryophyllene synthase activity may likewise apply in connection with the hybridizing polynucleotides mentioned herein.

In another aspect, the present invention relates to recombinant nucleic acid molecules comprising the polynucleotide of the invention described above. The term "recombinant nucleic acid molecule" refers to a nucleic acid molecule which contains in addition to a polynucleotide of the invention as described above at least one further heterologous coding or non-coding nucleotide sequence. The term "heterologous" means that said polynucleotide originates from a different species or from the same species, however, from another location in the genome than said added nucleotide sequence or is synthetic. The term "recombinant" implies that nucleotide sequences are combined into one nucleic acid molecule by the aid of human intervention. The recombinant nucleic acid molecule of the invention can be used alone or as part of a vector.
For instance, the recombinant nucleic acid molecule may encode the polypeptide having caryophyllene synthase activity fused to a marker sequence, such as a peptide, which facilitates purification of the fused polypeptide. The marker sequence may for example be a hexa-histidine peptide, such as the tag provided in a pQE vector (Qiagen, Inc.), the streptavidin tag of ASKIBA vectors (IBA GmbH, Göttingen, Germany) or the His tag used in Example 2 (see infra), which provide for convenient purification of the fusion polypeptide. Another suitable marker sequence may be the HA tag which corresponds to an epitope derived from influenza hemagglutinin polypeptide (Wilson, Cell 37 (1984), 767). As a further example, the marker sequence may be glutathione-S-transferase (GST) which, apart from providing a purification tag, enhances polypeptide stability, for instance, in bacterial expression systems.

In a preferred embodiment, the recombinant nucleic acid molecules further comprise expression control sequences operably linked to the polynucleotide comprised in the recombinant nucleic acid molecule. More preferably, these recombinant nucleic acid molecules are expression cassettes.
The term "operatively linked" or "operably linked", as used throughout the present description, refers to a linkage between one or more expression control sequences and the coding region in the polynucleotide to be expressed in such a way that expression is achieved under conditions compatible with the expression control sequence.
Expression comprises transcription of the heterologous DNA sequence, preferably into a translatable mRNA. Regulatory elements ensuring expression in prokaryotic as well as in eukaryotic cells, preferably in plant cells, are well known to those skilled in the art. They encompass promoters, enhancers, termination signals, targeting signals and the like. Examples are given further below in connection with explanations concerning vectors. In the case of eukaryotic cells, expression control sequences may comprise poly-A signals ensuring termination of transcription and stabilization of the transcript, for example, those of the 35S RNA from Cauliflower Mosaic Virus (CaMV) or the nopaline synthase gene from Agrobacterium tumefaciens. Additional regulatory elements may include transcriptional as well as translational enhancers. A plant translational enhancer often used is the CaMV omega sequence. Similarly, the inclusion of an intron (e.g. intron-1 from the shrunken gene of maize) has been shown to increase expression levels by up to 100-fold (Mait, Transgenic Research 6 (1997), 143-156; Ni, Plant Journal 7 (1995), 661-676).
Promoters for use in connection with the recombinant nucleic acid molecule of the invention may be homologous or heterologous with regard to its origin and/or with regard to the gene to be expressed. Suitable promoters are for instance the promoter of the 35S RNA of the Cauliflower Mosaic Virus (see for instance US-A 5,352,605) and the ubiquitin-promoter (see for instance US-A 5,614,399) which lend themselves to constitutive expression. However, promoters which are only activated at a point in time determined by external influences can also be used (see for instance WO 93/07279). In this connection, promoters of heat shock proteins which permit simple induction may be of particular interest. Likewise, artificial and/or chemically inducible promoters may be used in this context. In one embodiment, promoters which ensure constitutive expression are preferred. However, in another preferred embodiment, the polynucleotide may be operatively linked to a promoter which is inducible upon attack by a root-damaging insect.

In a preferred embodiment, the expression control sequences of the recombinant nucleic acid molecules comprise a root-specific promoter.
Suitable promoter sequences are known to the skilled person and are described in the pertinent literature. An example for such a sequence is the root-specific promotor of a glycolsyltransferase from Arabidopsis (Vijaybhaskar et al., 2008). Preferably, the root-specific promoter is constitutive or inducible upon attack by a root-damaging insect. Particularly preferred promoters are the maize caryophyllene promoter described in WO 2008/006564 and the maize ubiquitin promoter used in Example 3.

Moreover, the invention relates to vectors, in particular plasmids, cosmids, viruses, bacteriophages and other vectors commonly used in genetic engineering, which contain the above-described polynucleotides or recombinant nucleic acid molecules of the invention. In a preferred embodiment, the vectors of the invention are suitable for the transformation of fungal cells, cells of microorganisms such as yeast or bacterial cells, animal cells or, in particular, plant cells. In a particularly preferred embodiment such vectors are suitable for stable transformation of plants.

In another preferred embodiment, the vectors further comprise expression control sequences operably linked to said polynucleotides contained in the vectors. These expression control sequences may be suited to ensure transcription and synthesis of a translatable RNA in prokaryotic or eukaryotic cells.
The expression of the polynucleotides of the invention in prokaryotic or eukaryotic cells, for instance in *Escherichia coli,* is interesting because it permits a more precise characterization of the biological activities of the encoded polypeptide. In particular, it is possible to express these polypeptides in such prokaryotic or eukaryotic cells which are free from interfering polypeptides. In addition, it is possible to insert different mutations into the polynucleotides by methods usual in molecular biology (see for instance Sambrook and Russell (2001), Molecular Cloning: A Laboratory Manual, CSH Press, Cold Spring Harbor, NY, USA), leading to the synthesis of polypeptides possibly having modified biological properties. In this regard it is on the one hand possible to produce deletion mutants in which polynucleotides are produced by progressive deletions from the 5' or 3' end of the coding DNA sequence, and said polynucleotides lead to the synthesis of correspondingly shortened polypeptides.
On the other hand, the introduction of point mutations is also conceivable at positions at which a modification of the amino acid sequence for instance influences the biological activity or the regulation of the polypeptide.
Moreover, mutants possessing a modified substrate or product specificity can be prepared. Furthermore, it is possible to prepare mutants having a modified activity-temperature-profile. Preferably, such mutants show an increased activity. Alternatively, mutants can be prepared the catalytic activity of which is abolished without losing substrate binding activity.
Furthermore, in the case of expression in plants, plant tissue or plant cells, the introduction of mutations into the polynucleotides of the invention allows the gene expression rate and/or the activity of the polypeptides encoded by the polynucleotides of the invention to be reduced or increased.
For genetic engineering in prokaryotic cells, the polynucleotides of the invention or parts of these molecules can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, *in vitro* mutagenesis, "primer repair", restriction or ligation can be used. In general, a sequence analysis, restriction analysis and other methods of biochemistry and molecular biology are carried out as analysis methods.
Additionally, the present invention relates to a method for producing genetically engineered host cells comprising introducing the above-described polynucleotides, recombinant nucleic acid molecules or vectors of the invention into a host cell.

Another embodiment of the invention relates to host cells, in particular prokaryotic or eukaryotic cells, genetically engineered with the above-described polynucleotides, recombinant nucleic acid molecules or vectors of the invention or obtainable by the above-mentioned method for producing genetically engineered host cells, and to cells derived from such transformed cells and containing a polynucleotide, recombinant nucleic acid molecule or vector of the invention. In a preferred embodiment the host cell is genetically modified in such a way that it contains the polynucleotide stably integrated into the genome. Preferentially, the host cell of the invention is a bacterial, yeast, fungus, plant or animal cell.
More preferably the polynucleotide can be expressed so as to lead to the production of a polypeptide having caryophyllene synthase activity. An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). An overview of yeast expression systems is for instance given by Hensing et al. (Antonie van Leuwenhoek 67 (1995), 261-279), Bussineau et al. (Developments in Biological Standardization 83 (1994), 13-19), Gellissen et al. (Antonie van Leuwenhoek 62 (1992), 79-93, Fleer (Current Opinion in Biotechnology 3 (1992), 486-496), Vedvick (Current Opinion in Biotechnology 2 (1991), 742-745) and Buckholz (Bio/Technology 9 (1991), 1067-1072).
Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E. coli, S. cerevisiae)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.
The transformation of the host cell with a polynucleotide or vector according to the invention can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc. The polypeptide according to the present invention can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Polypeptide refolding steps can be used, as necessary, in completing configuration of the polypeptide. Finally, high performance liquid chromatography (HPLC) can be employed for final purification steps.

Accordingly, the present invention also relates to a method for the production of a polypeptide encoded by a polynucleotide of the invention as described above in which the above-mentioned host cell is cultivated under conditions allowing for the expression of the polypeptide and in which the polypeptide is isolated from the cells and/or the culture medium.

Moreover, the invention relates to a polypeptide which is encoded by a polynucleotide according to the invention or obtainable by the above-mentioned method for the production of a polypeptide.
The polypeptide of the present invention may, e.g., be a naturally purified product or a product of chemical synthetic procedures or produced by recombinant techniques from a prokaryotic or eukaryotic host (for example, by bacterial, yeast, higher plant, insect and mammalian cells in culture). Depending upon the host employed in a recombinant production procedure, the polypeptide of the present invention may be glycosylated or may be non-glycosylated. The polypeptide of the invention may also include an initial methionine amino acid residue. The polypeptide according to the invention may be further modified to contain additional chemical moieties not normally part of the polypeptide. Those derivatized moieties may, e.g., improve the stability, solubility, the biological half life or absorption of the polypeptide. The moieties may also reduce or eliminate any undesirable side effects of the polypeptide and the like. An overview for these moieties can be found, e.g., in Remington's Pharmaceutical Sciences (18th ed., Mack Publishing Co., Easton, PA (1990)). Polyethylene glycol (PEG) is an example for such a chemical moiety which has been used for the preparation of therapeutic polypeptides. The attachment of PEG to polypeptides has been shown to protect them against proteolysis (Sada et al., J. Fermentation Bioengineering 71 (1991), 137-139). Various methods are available for the attachment of certain PEG moieties to polypeptides (for review see: Abuchowski et al., in "Enzymes as Drugs"; Holcerberg and Roberts, eds. (1981), 367-383). Generally, PEG molecules are connected to the polypeptide via a reactive group found on the polypeptide. Amino groups, e.g. on lysines or the amino terminus of the polypeptide are convenient for this attachment among others.

Furthermore, the present invention relates to a binding molecule specifically recognizing the polypeptide of the invention.

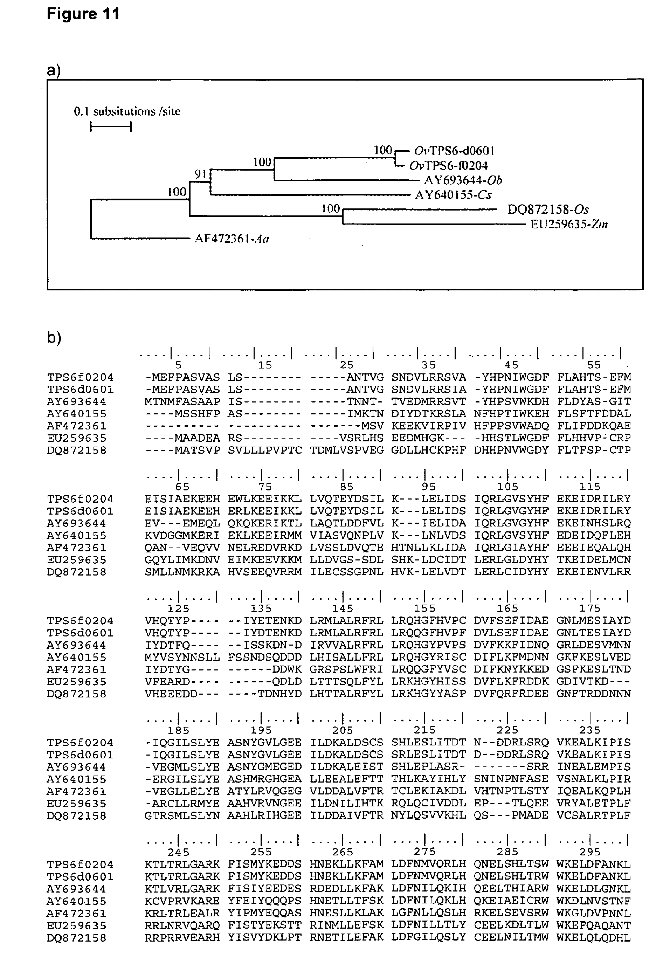

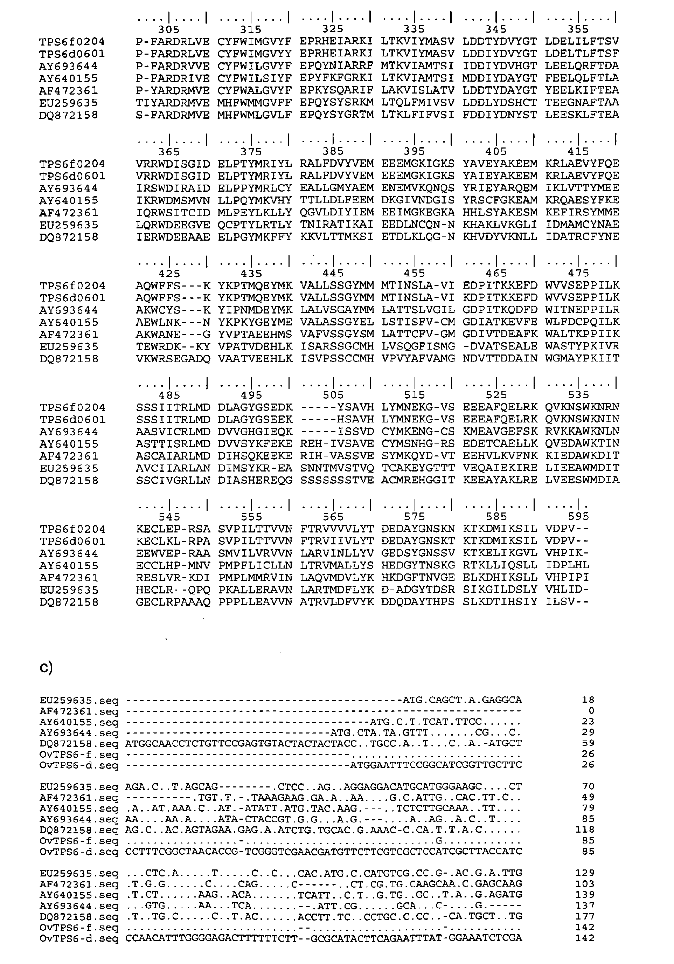

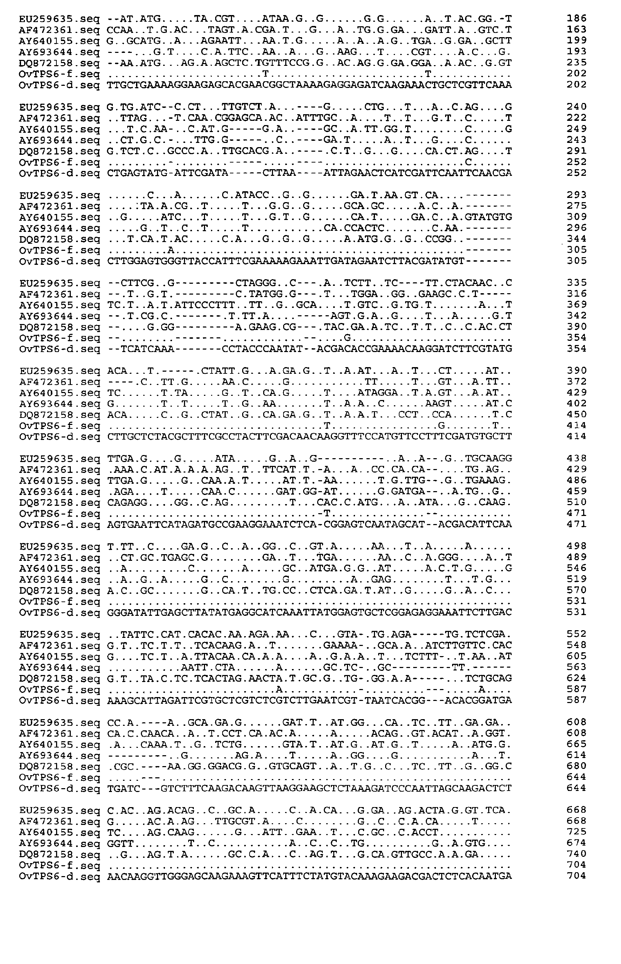

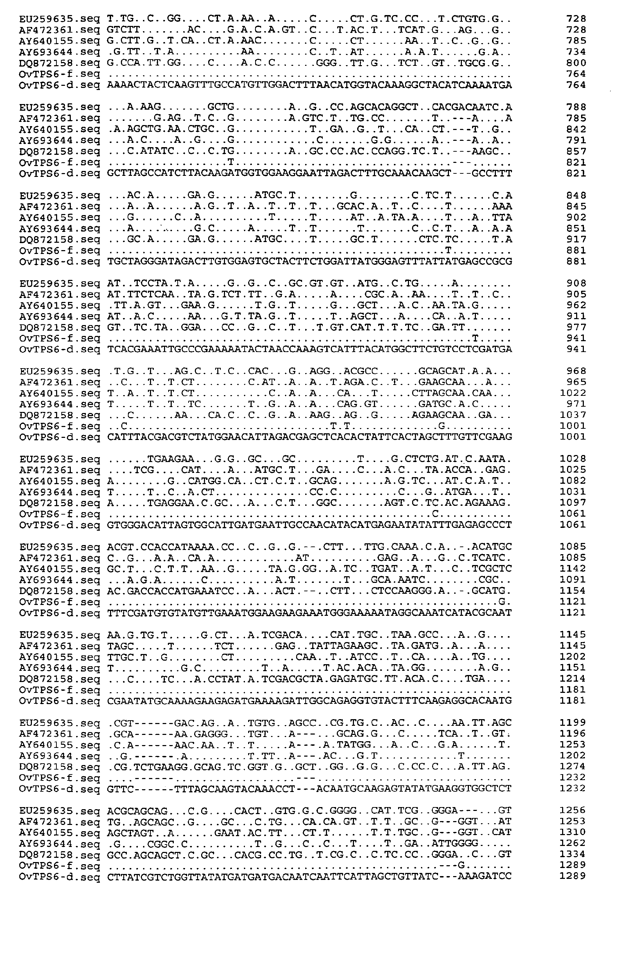

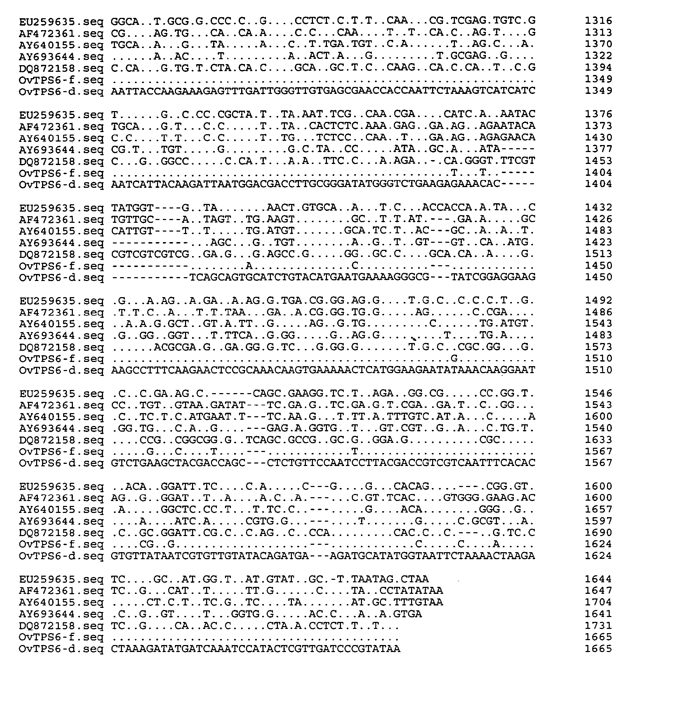

The term "specifically recognizing" is meant to refer to the high affinity antibodies or other binding molecules known in the prior art typically have for the target molecule against which they were prepared.
Advantageously, the term "specifically recognizing" refers to a specificity of the binding molecule that allows a distinction between the polypeptide of the invention and related terpene synthase polypeptides, in the sense that the binding molecule does not show a significant cross-reactivity with the latter ones. Said related terpene synthase polypeptides may include the caryophyllene synthases known from dicotyledonous plants and/or other terpene synthases, especially caryophyllene synthase. The person skilled in the art is able to prepare such distinctive binding molecules, for example by selecting non-conserved amino acid stretches, e.g. on the basis of an alignment such as the one shown in Figure 11, and using them as a starting point (e.g. as an antigen in the case of antibodies) for preparing the binding molecule.
The binding molecule of the present invention may be selected form the group consisting of antibodies, affybodies, trinectins, anticalins, aptamers, RNAs, PNAs and the like, whereby antibodies are preferred.
Based on prior art literature, the person skilled in the art is familiar with obtaining specific binding molecules that may be useful in the methods, kits and uses provided herein. These molecules are directed and bind specifically to or specifically label the polypeptide of the invention described herein. Non-limiting examples of suitable binding molecules may be selected from aptamers (Gold, Ann. Rev. Biochem. 64 (1995), 763-797)), aptazymes, RNAi, shRNA, RNAzymes, ribozymes (see e.g., EP-B1 0 291 533, EP-A1 0 321 201, EP-B1 0 360 257), antisense DNA, antisense oligonucleotides, antisense RNA, siRNA, antibodies (Harlow and Lane "Antibodies, A Laboratory Manual", CSH Press, Cold Spring Harbor, 1988), affibodies (Hansson, lmmunotechnology 4 (1999), 237-252; Henning, Hum Gene Ther. 13 (2000), 1427-1439), lectins, trinectins (Phylos Inc., Lexington, Massachusetts, USA; Xu, Chem. Biol. 9 (2002), 933), anticalins (EPB1 1 017 814) and the like. In accordance with the present invention, the term "aptamer" means nucleic acid molecules that can specifically bind to target molecules. Aptamers commonly comprise RNA, single stranded DNA, modified RNA or modified DNA molecules. The preparation of aptamers is well known in the art and may involve, inter alia, the use of combinatorial RNA libraries to identify binding sites (Gold (1995), Ann. Rev. Biochem 64 , 763-797).
A preferred binding molecule in the context of the present invention is an antibody specific for the polypeptide of the present invention.
The antibody useful in context of the present invention can be, for example, polyclonal or monoclonal. The term "antibody" also comprises derivatives or fragments thereof which still retain the binding specificity.
The polypeptide according to the invention, its fragments or other derivatives thereof, or cells expressing them can be used as an immunogen to produce antibodies thereto. The present invention in particular also includes chimeric, single chain, and humanized antibodies, as well as Fab fragments, or the product of an Fab expression library. Various procedures known in the art may be used for the production of such antibodies and fragments.
In context of the present invention, the term "antibody" relates to full immunoglobulin molecules as well as to parts of such immunoglobulin molecules substantially retaining binding specificity. Furthermore, the term relates, as discussed above, to modified and/or altered antibody molecules, like chimeric and humanized antibodies. The term also relates to recombinantly or synthetically generated/synthesized antibodies. The term also relates to intact antibodies as well as to antibody fragments thereof, like, separated light and heavy chains, Fab, Fab/c, Fv, Fab', F(ab')₂. The term "antibody" also comprises bifunctional antibodies, trifunctional antibodies and antibody constructs, like single chain Fvs (scFv) or antibody-fusion proteins.
Techniques for the production of antibodies are well known in the art and described, e.g. in Harlow and Lane "Antibodies, A Laboratory Manual", CSH Press, Cold Spring Harbor, 1988. Antibodies directed against a polypeptide according to the present invention can be obtained, e.g., by direct injection of the polypeptide into an animal or by administering the polypeptide to an animal, preferably a non-human animal. The antibody so obtained will then bind the polypeptide itself. In this manner, even a sequence encoding only a fragment of the polypeptide can be used to generate antibodies binding the whole native polypeptide.
Particularly preferred in the context of the present invention are monoclonal antibodies. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique
(Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Various procedures are known in the art and may be used for the production of such antibodies and/or fragments. Thus, the antibody derivatives can also be produced by peptidomimetics. Further, techniques described for the production of single chain antibodies (see, inter alia, US Patent 4,946,778) can be adapted to produce single chain antibodies specifically recognizing the polypeptide of the invention. Also, transgenic animals may be used to express humanized antibodies to the polypeptide of the invention.
Surface plasmon resonance as employed in the BlAcore system can be used to increase the efficiency of phage antibodies which bind to an epitope of the polypeptide of the invention (Schier, Human Antibodies Hybridomas 7 (1996), 97-105; Malmborg, J. Immunol. Methods 183 (1995), 7-13). Accordingly, also phage antibodies can be used in context of this invention.
Binding molecules according to the invention can be used for detecting the presence, absence or amount of the polypeptide of the invention in a sample, in particular in the framework of methods and uses described herein further below. The binding molecules may furthermore be used for isolating the polypeptide from a biological source material or for detecting the polypeptide in a sample.

Furthermore, the present invention relates to a method for producing a transgenic plant, plant cell or plant tissue comprising the introduction of at least one of the above-described polynucleotides, recombinant nucleic acid molecules or vectors of the invention into the genome of a plant, plant cell or plant tissue.
Preferably, said method comprises (a) the introduction of at least one of said polynucleotides, recombinant nucleic acid molecules or vectors into the genome of a plant cell and regenerating the cell of (a) to a transgenic plant or transgenic plant tissue. Optionally, the method may further comprise step (c) producing progeny from the plants produced in step (b).

In one aspect, the present invention accordingly refers to transgenic plant cells which are genetically engineered with at least one of the polynucleotides, recombinant nucleic acid molecules or vectors of the invention described above or of transgenic plant cells which are obtainable by the aforementioned method for producing a transgenic plant cell.

In a further aspect, the invention relates to transgenic plants or plant tissue comprising plant cells which are genetically engineered with the polynucleotide of the invention or which contain the recombinant nucleic acid molecule or the vector of the invention or to transgenic plants obtainable by the method mentioned above.
Preferably, in the transgenic plant of the invention, the polynucleotide of the invention is expressed at least in one part, i.e. organ, tissue or cell type, of the plant. Preferably, this expression leads to an increase of caryophyllene synthase activity in the cells or tissues which express said polynucleotide. Increase of activity can be detected for instance by measuring the amount of transcript and/or protein and/or terpene emission in the transformed cell, tissue or plant in comparison to corresponding measurements at non-transformed plant cells, tissue or plants. According to the teachings of the present invention, an increase of the activity of the polypeptide of the invention in transgenic plants leads to an increase of resistance against a root-damaging insect to which a corresponding wild-type plant is susceptible or at least more susceptible.
The term "increased activity" refers to a significant increase of the caryophyllene synthase activity of the polypeptide of the invention in the transgenic plant compared to a corresponding wild-type plant. Preferably, said activity is increased in the transgenic plant by at least 10%, preferably by at least 20%, more preferably by at least 50%, and even more preferred by at least 100% as compared to the corresponding wild-type plant. It is even more preferred that the corresponding wild-type plant shows no detectable caryophyllene synthase activity as opposed to a significant caryophyllene synthase activity observable in the transgenic plant of the invention. Caryophyllene synthase activity may be determined by the use of enzyme assays using a preparation from a plant sample or measurement of volatile emissions of the plant by applying suitable methods known in the art or described herein.
An increase of the activity of the polypeptide of the invention may also be inferred from a significant increase of the amount of corresponding transcript and/or protein present in the transgenic plant. Preferentially, transgenic plants having an increased activity of the polypeptide of the invention may be characterized by an increase of the amount of transcript corresponding to the polynucleotide of the invention by at least 20%, preferably at least 50% and more preferably at least 100% as compared to the corresponding wild-type plant. Likewise, it is preferred that transgenic plants having an increased activity of the polypeptide of the invention may be characterized by an increase of the protein amount of the polypeptide of the invention by at least 20%, preferably at least 50% and more preferably at least 100% as compared to the corresponding wild-type plant. Most preferably, the corresponding wild-type plant shows no detectable amount of such transcript and/or such protein as opposed to significant transcript and/or protein amounts observable in the transgenic plant of the invention.
The polynucleotide introduced into the transgenic plant can in principle be expressed in all or substantially all cells of the plant. However, it is also possible that it is only expressed in certain parts, organs, cell types, tissues etc., provided that expression in the root is ensured. Moreover, it is possible that expression of the polynucleotide only takes place upon induction, at a certain developmental stage or, as it is preferred, upon induction by attack of the root by a root-damaging insect. In a preferred embodiment, the polynucleotide is expressed in those parts of the plant that are exposed to pathogen attack, for example the rhizodermis.
In order to be expressed, the polynucleotide that is introduced into a plant cell is preferably operatively linked to one or more expression control sequences, e.g. a promoter, active in this plant cell. Suitable promoter sequences are known to the skilled person and are described in the pertinent literature.
The promoter may be homologous or heterologous with regard to its origin and/or with regard to the gene to be expressed. Suitable promoters are for instance the promoter of the 35S RNA of the Cauliflower Mosaic Virus (see for instance US-A 5,352,605) and the ubiquitin-promoter (see for instance US-A 5,614,399) which lend themselves to constitutive expression. However, promoters which are only activated at a point in time determined by external influences can also be used (see for instance WO 93/07279). In this connection, promoters of heat shock proteins which permit simple induction may be of particular interest. Likewise, artificial and/or chemically inducible promoters may be used in this context. In one embodiment, promoters which ensure constitutive expression are preferred. However, in another preferred embodiment, the polynucleotide may be operatively linked to a promoter which is inducible upon attack by a root-damaging insect. Furthermore preferred is the use of a promoter that is specific for expression in the root, preferably such a promoter is constitutive or inducible upon attack by a root-damaging insect. Particularly preferred promoters are the maize caryophyllene promoter described in WO 2008/006564 and the maize ubiquitin promoter used in Example 3.
Moreover, the polynucleotide may be linked to a termination sequence which serves to terminate transcription correctly and to add a poly-A-tail to the transcript which is believed to have a function in the stabilization of the transcripts. Such elements are described in the literature (see for instance Gielen et al., EMBO J. 8 (1989), 23-29) and can be replaced at will.
Furthermore, if needed, polypeptide expression can in principle be targeted to any sub-localization of plant cells (e.g. cytosol, plastids, vacuole, mitochondria) or the plant (e.g. apoplast). In order to achieve the localization in a particular compartment, the coding region to be expressed may be linked to DNA sequences encoding a signal sequence (also called "transit peptide") ensuring localization in the respective compartment. It is evident that these DNA sequences are to be arranged in the same reading frame as the coding region to be expressed. Preferred in connection with the present invention is the expression of the polynucleotide of the invention into the cytosol and/or the endoplasmatic reticulum since this is presumed to be the site of sesquiterpene synthesis in plant cells (Chen, Plant Cell 15 (2003), 481-494).
In order to ensure the location in the plastids, it is conceivable to use one of the following transit peptides: of the plastidic Ferredoxin: NADP+ oxidoreductase (FNR) of spinach which is enclosed in Jansen et al. (Current Genetics 13 (1988), 517-522). In particular, the sequence ranging from nucleotides -171 to 165 of the cDNA sequence disclosed therein can be used which comprises the 5' non-translated region as well as the sequence encoding the transit peptide. Another example is the transit peptide of the waxy protein of maize including the first 34 amino acid residues of the mature waxy protein (Klösgen et al., Mol. Gen. Genet. 217 (1989), 155-161). It is also possible to use this transit peptide without the first 34 amino acids of the mature protein. Furthermore, the signal peptides of the ribulose bisphosphate carboxylase small subunit (Wolter et al., Proc. Natl. Acad. Sci. USA 85 (1988), 846-850; Nawrath et al., Proc. Natl. Acad. Sci. USA 91 (1994), 12760-12764), of the NADP malate dehydrogenase (Gallardo et al., Planta 197 (1995), 324-332), of the glutathione reductase (Creissen et al., Plant J. 8 (1995), 167-175) or of the R1 protein (Lorberth et al. Nature Biotechnology 16, (1998), 473-477) can be used.

In order to ensure the location in the vacuole, it is conceivable to use one of the following transit peptides: the N-terminal sequence (146 amino acids) of the patatin protein (Sonnewald et al., Plant J. 1 (1991), 95-106) or the signal sequences described by Matsuoka and Neuhaus (Journal of Experimental Botany 50 (1999), 165-174); Chrispeels and Raikhel (Cell 68 (1992), 613-616); Matsuoka and Nakamura (Proc. Natl. Acad. Sci. USA 88 (1991), 834-838); Bednarek and Raikhel (Plant Cell 3 (1991), 1195-1206); and Nakamura and Matsuoka (Plant Phys. 101 (1993), 1-5).
In order to ensure the localization in the mitochondria, it is for example conceivable to use the transit peptide described by Braun (EMBO J. 11, (1992), 3219-3227).
In order to ensure the localization in the apoplast, it is conceivable to use one of the following transit peptides: signal sequence of the proteinase inhibitor II-gene (Keil et al., Nucleic Acid Res. 14 (1986), 5641-5650; von Schaewen et al., EMBO J. 9 (1990), 30-33), of the levansucrase gene from Erwinia amylovora (Geier and Geider, Phys. Mol. Plant Pathol. 42 (1993), 387-404), of a fragment of the patatin gene B33 from Solanum tuberosum, which encodes the first 33 amino acids (Rosahl et al., Mol Gen. Genet. 203 (1986), 214-220) or of the one described by Oshima et al. (Nucleic Acid Res. 18 (1990), 181).
The transgenic plants of the invention may, in principle, be plants of any plant species. They may be both monocotyledonous or dicotyledonous plants. Preferably, the plants are useful plants, i.e. commercially important plants, cultivated by man for nutrition or for technical, in particular industrial, purposes. They may be sugar-storing and/or starch-storing plants, for instance cereal species (rye, barley, oat, wheat, maize, millet, sago etc.), rice, pea, marrow pea, cassava, sugar cane, sugar beet and potato; tomato, rape, soybean, hemp, flax, sunflower, cow pea or arrowroot, fiber-forming plants (e.g. flax, hemp, cotton), oil-storing plants (e.g. rape, sunflower, soybean) and protein-storing plants (e.g. legumes, cereals, soybeans). The plants within the scope of the invention also include fruit trees, palms and other trees or wooden plants being of economical value such as in forestry. Moreover, the method of the invention relates to forage plants (e.g. forage and pasture grasses, such as alfalfa, clover, ryegrass) and vegetable plants (e.g. tomato, lettuce, chicory) and ornamental plants (e.g. roses, tulips, hyacinths). Preferably, the transgenic plant of the invention is a monocotyledonous plant, more preferably a plant of the Poaceae family, particularly preferably a wheat, barley, oat, rye, rice or sorghum plant. Most preferred are transgenic plants being maize, in particular maize plants which, in the wild-type state, do not express an active caryophyllene synthase (Köllner et al., 2008).

According to the provisions of the invention, transgenic plants can be prepared by introducing a polynucleotide into plant cells and regenerating the transformed cells to plants by methods well known to the person skilled in the art.
Methods for the introduction of foreign genes into plants are also well known in the art. These include, for example, the transformation of plant cells or tissues with T-DNA using *Agrobacterium tumefaciens* or *Agrobacterium rhizogenes,* the fusion of protoplasts, direct gene transfer (see, e.g., EP-A 164 575), injection, electroporation, vacuum infiltration, biolistic methods like particle bombardment, pollen-mediated transformation, plant RNA virus-mediated transformation, liposome-mediated transformation, transformation using wounded or enzyme-degraded immature embryos, or wounded or enzyme-degraded embryogenic callus and other methods known in the art. The vectors used in the method of the invention may contain further functional elements, for example "left border"- and "right border"-sequences of the T-DNA of *Agrobacterium* which allow stable integration into the plant genome. Furthermore, methods and vectors are known to the person skilled in the art which permit the generation of marker free transgenic plants, i.e. the selectable or scorable marker gene is lost at a certain stage of plant development or plant breeding. This can be achieved by, for example co-transformation (Lyznik, Plant Mol. Biol. 13 (1989), 151-161; Peng, Plant Mol. Biol. 27 (1995), 91-104) and/or by using systems which utilize enzymes capable of promoting homologous recombination in plants (see, e.g., WO97/08331; Bayley, Plant Mol. Biol. 18 (1992), 353-361); Lloyd, Mol. Gen. Genet. 242 (1994), 653-657; Maeser, Mol. Gen. Genet. 230 (1991), 170-176; Onouchi, Nucl. Acids Res. 19 (1991), 6373-6378). Methods for the preparation of appropriate vectors are described by, e.g., Sambrook and Russell (2001), Molecular Cloning: A Laboratory Manual, CSH Press, Cold Spring Harbor, NY, USA.
Suitable strains of *Agrobacterium tumefaciens* and vectors as well as transformation of *Agrobacteria* and appropriate growth and selection media are well known to those skilled in the art and are described in the prior art (GV3101 (pMK90RK), Koncz, Mol. Gen. Genet. 204 (1986), 383-396; C58C1 (pGV 3850kan), Deblaere, Nucl. Acid Res. 13 (1985), 4777; Bevan, Nucleic. Acid Res. 12(1984), 8711; Koncz, Proc. Natl. Acad. Sci. USA 86 (1989), 8467-8471; Koncz, Plant Mol. Biol. 20 (1992), 963-976; Koncz, Specialized vectors for gene tagging and expression studies. In: Plant Molecular Biology Manual Vol 2, Gelvin and Schilperoort (Eds.), Dordrecht, The Netherlands: Kluwer Academic Publ. (1994), 1-22; EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46; An, EMBO J. 4 (1985), 277-287). Although the use of *Agrobacterium tumefaciens* is preferred in the method of the invention, other *Agrobacterium* strains, such as *Agrobacterium rhizogenes,* may be used, for example if a phenotype conferred by said strain is desired.
Methods for the transformation using biolistic methods are well known to the person skilled in the art; see, e.g., Wan, Plant Physiol. 104 (1994), 37-48; Vasil, Bio/Technology 11 (1993), 1553-1558 and Christou (1996) Trends in Plant Science 1, 423-431. Microinjection can be performed as described in Potrykus and Spangenberg (eds.), Gene Transfer To Plants. Springer Verlag, Berlin, NY (1995).
The transformation of most dicotyledonous plants is possible with the methods described above. But also for the transformation of monocotyledonous plants several successful transformation techniques have been developed. These include the transformation using biolistic methods as, e.g., described above as well as protoplast transformation, electroporation of partially permeabilized cells, introduction of DNA using glass fibers, etc. Also, the transformation of monocotyledonous plants by means of Agrobacterium-based vectors has been described (Chan et al., Plant Mol. Biol. 22 (1993), 491-506; Hiei et al., Plant J. 6 (1994) 271-282; Deng et al, Science in China 33 (1990), 28-34; Wilmink et al, Plant Cell Reports 11 (1992), 76-80; May et al., Bio/Technology 13 (1995), 486-492; Conner and Dormisse, Int. J. Plant Sci. 153 (1992), 550-555; Ritchie et al. Transgenic Res. 2 (1993), 252-265). An alternative system for transforming monocotyledonous plants is the transformation by the biolistic approach (Wan and Lemaux, Plant Physiol. 104 (1994), 37-48; Vasil et al., Bio/Technology 11 (1993), 1553-1558; Ritala et al., Plant Mol. Biol. 24 (1994) 317-325; Spencer et al., Theor. Appl. Genet. 79 (1990), 625-631). The transformation of maize in particular has been repeatedly described in the literature (see for instance WO 95/06128, EP 0 513 849, EP 0 465 875, EP 29 24 35; Fromm et al, Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet. 80, (1990), 721-726; and, preferably, The Maize Handbook. Eds. M. Freeling and V. Walbot, Springer Verlag New York, 1996). The successful transformation of other types of cereals has also been described for instance of barley (Wan and Lemaux, supra; Ritala et al., supra, Krens et al., Nature 296 (1982), 72-74), wheat (Nehra et al., Plant J. 5 (1994), 285-297) and rice.
The resulting transformed plant cell can then be used to regenerate a transformed plant in a manner known by a skilled person.

In addition, the present invention relates to transgenic plants which show an increased activity of the polypeptide encoded by the polynucleotide the invention compared to a corresponding wild-type plant.
In this context, the "increased activity" complies with the definition stated above and it can be determined accordingly.
Corresponding increases of the activity of the polypeptide of the invention may for instance be achieved by expressing said polynucleotide in cells of a transgenic plant from a heterologous construct for example as described above. However, the state of the art provides further methods for achieving a corresponding increased activity. For example, the endogenous gene encoding the caryophyllene synthase of the invention may be modified accordingly at its natural location, e.g. by homologous recombination. In particular, the promoter of this gene can for instance be altered in a way that promoter activity is enhanced. In the alternative, the coding region of the gene can be modified so that the encoded polypeptide shows an increased activity, e.g. by specifically substituting amino acid residues so as to re-establish the amino acid sequence encoded by an active allele, such as the alleles characterized by the coding sequences of SEQ ID Nos. 1 and 3. Applicable homologous recombination techniques (also known as *"in vivo* mutagenesis") are known to the person skilled in the art and are described in the literature. One such technique involves the use of a hybrid RNA-DNA oligonucleotide ("chimeroplast") which is introduced into cells by transformation (TIBTECH 15 (1997), 441-447; WO95/15972; Kren, Hepatology 25 (1997), 1462-1468; Cole-Strauss, Science 273 (1996), 1386-1389). Thereby, part of the DNA component of the RNA-DNA oligonucleotide is homologous with the target gene sequence, however, displays in comparison to this sequence a mutation or a heterologous region which is surrounded by the homologous regions. The term "heterologous region" refers to any sequence that can be introduced and which is different from that to be modified. By means of base pairing of the homologous regions with the target sequence followed by a homologous recombination, the mutation or the heterologous region contained in the DNA component of the RNA-DNA oligonucleotide can be transferred to the corresponding gene. By means of *in vivo* mutagenesis, any part of the gene encoding the caryophyllene synthase of the invention can be modified as long as it results in an increase of the activity of this protein.

In a preferred embodiment, the above-described transgenic plants show, upon an increased activity of the protein encoded by the polynucleotide of the invention, an increased resistance against a root-damaging insect to which a corresponding wild-type plant is more susceptible.
The term "increased resistance" may refer both to an enhancement of a resistance already present in the wild-type plant and to the establishment of a resistance that is not present in the wild-type plant.
Preferably, these transgenic plants contain a polynucleotide as defined above, i.e. a polynucleotide or a recombinant nucleic acid molecule that is introduced in a plant cell and the presence of which in the genome of said plant preferably leads to an increased activity of the caryophyllene synthase of the invention, stably integrated into the genome.

The invention also relates to propagation material of the transgenic plants of the invention, said material comprising plant cells according to the invention. The term "propagation material" comprises those components or parts of the plant which are suitable to produce offspring vegetatively or generatively. Suitable means for vegetative propagation are for instance cuttings, callus cultures, rhizomes or tubers. Other propagation material includes for instance fruits, seeds, seedlings, protoplasts, cell cultures etc. The preferred propagation materials are tubers and seeds.
The invention also relates to harvestable parts of the plants of the invention such as, for instance, fruits, seeds, tubers, rootstocks, leaves or flowers.

In accordance with the above explanations, the invention furthermore relates to a method for conferring resistance or increased resistance against a root-damaging insect to a plant comprising the step of providing a transgenic plant in which the activity of the polypeptide encoded by the above-described polynucleotide of the invention is increased as compared to a corresponding wild-type plant.

Also within the scope of the invention is the use of the polynucleotide, of the recombinant nucleic acid molecule, of the vector, of the host cell, of the polypeptide, of the binding molecule or of the transgenic plant of the invention, said matters being described above in detail, for establishing or enhancing resistance against a root-damaging insect in plants.
In a preferred embodiment of the above-mentioned method and the above-mentioned use, the plants are maize plants and the root-damaging insect is the corn rootworm.

Moreover, the present invention relates to the use of the transgenic plant of the invention or of propagation material thereof together with entomopathogenic nematodes for growing said plant. Preferably, said transgenic plant or plant is maize, said propagation material is from a maize plant and such entomopathoganic nematode is *Heterorhabditis megidis.*
It is envisaged that the biological control accomplished by the attraction of entomopathogenic nematodes upon insect attack through the release of caryophyllene may be improved by adding the nematodes to the soil. The nematodes can be cultivated according to methods described in the prior art literature and known to the skilled person. Advantageously, the nematodes are deployed to the field when, or shortly before, attack by root-damaging insects can be expected. Alternatively, the nematodes can be deployed at a time relatively long before attack, for example together with sowing, since nematodes are able to persist for longer periods without nutrition or can feed on other insects present in the soil.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.google.de. An overview of patent information in biotechnology and a survey of relevant sources of patent information useful for retrospective searching and for current awareness is given in Berks, TIBTECH 12 (1994), 352-364.
Furthermore, the term "and/or" when occurring herein includes the meaning of "and", "or" and "all or any other combination of the elements connected by said term".

The present invention is further described by reference to the following non-limiting figures and examples.

The Figures show:
- **Figure 1**: shows a dendrogram of monoterpene and sesquiterpene synthases from *O*. *vulgare* and functionally related terpene synthases mainly from other species of Lamiaceae. The dendrogram was constructed using the nearest neighbour-joining method (Clustal X method) and visualized with Treecon 1.3b. *Aa - Artemisia annua:* (*E*)-β-caryophyllene synthase (AF472361); *Cl Citrus limon*: γ-terpinene synthase (AF514286); *Mc - Mentha citrata*: linalool synthase (AY083653); *Ob - Ocimum basilicum:* fenchol synthase (AY693648), myrcene synthase (AY693649), selinene synthase (AY693643), germacrene D synthase (AY693644); *Ov - Origanum vulgare:* sabinene synthase, γ-terpinene synthase 1, (-)- germacrene D synthase, bicyclo-germacrene synthase, γ-terpinene synthase 2, (*E*)-β-caryophyllene synthase and (*E*)-β-ocimene synthase; *Pf - Perilla frutescens:* geraniol synthase (DQ088667), linalool synthase (DQ234300); *Pfc - Perilla frutescens* var. *crispa:* linalool synthase (AF444798); *Pc - Perilla citriodora:* linalool synthase (AF917193); *Ro - Rosmarinus officinalis:* bornyl synthase (EF495245); *So - Salvia officinalis:* sabinene synthase (AF051901). GenBank accession numbers in parentheses.
- **Figure 2**: shows a GC-MS chromatogram for (*E*)-β-caryophyllene synthase *Ov*TPS6. Assays were conducted with crude protein extracts from heterologous expression in *E. coli* with farnesyl diphosphate (FPP) as substrate. Volatiles were absorbed to a solid phase micro extraction fiber. Products: 14, (*E*)-β-caryophyllene and 15, α-humulene.

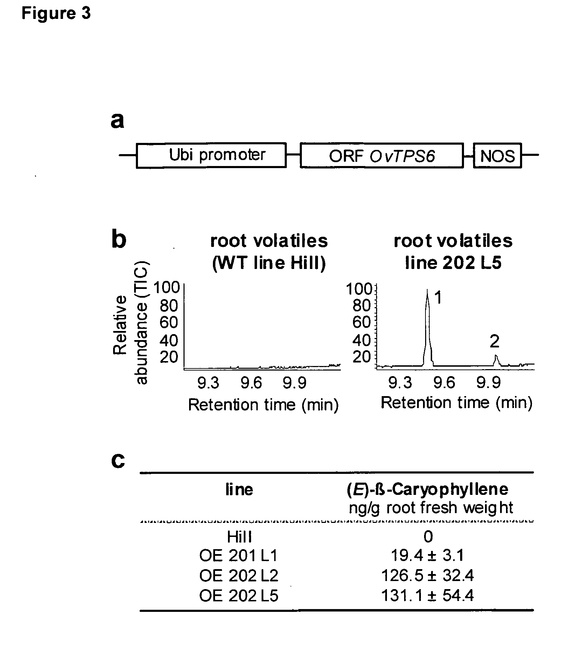

- **Figure 3**: shows that the insertion of the (*E*)-β-caryophyllene synthase gene TPS6 from *Origanum vulgare* in maize variety Hill results in a constitutive production of (*E*)-β**-**caryophyllene (EβC). (a) Terpene synthase overexpression construct. (b) A typical chromatogram obtained for the volatiles emitted by roots of the hybrid variety Hill line alongside a chromatogram for one of the transformed lines. Peak 1 is EβC and peak 2 is α-humulene, a side product of (*E*)-β-caryophyllene synthase. (c) Average quantities of EβC present in the roots of the untransformed Hill and the three transformed lines that were used in the field experiments (n=8).

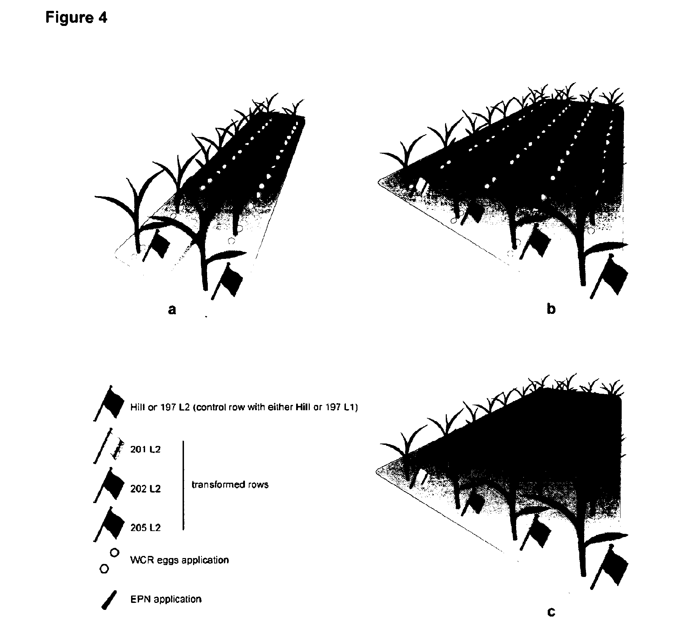

- **Figure 4**: shows the design of the field plots where transformed lines were tested alongside control lines. Plants were grown in plots with two or four rows with eight plants per row. Four plants of each row were used for the root damage rating and the four remaining plants received emergence cages to monitor emergence of WCR adult. (a) One row of a control line was planted alongside a row of a transformed maize line. Each plant was infested with WCR eggs and entomopathogenic nematode (EPN) were applied in a trench in the middle of the two rows (n=30). (b) As a first control plot, a row of eight control plants was planted alongside three rows each with eight plants of a transformed line. These plants were also infested with WCR eggs, but no EPN were applied (n=5). (c) The second type of control plot design was the same as for (b), but no WCR eggs or EPN were applied (n=5).

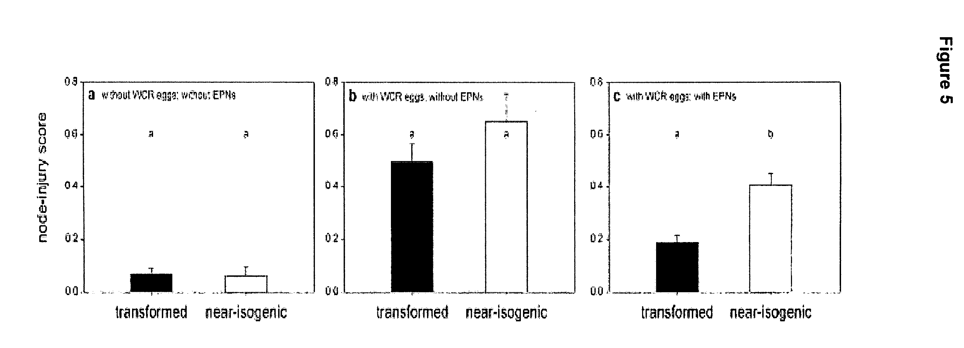

- **Figure 5**: shows that the average root damage on transformed plants was lower in plots that were treated with EPN. (a) Root damage measured from plants in the control plots that had received neither WCR eggs nor *H. megidis* was minimal and there was no difference between transformed and non-transformed plants (n = 5, P=0.88). (b) Root damage in control plots that only received WCR eggs but no nematodes was substantial. Again, no significant difference was found between the transformed and non-transformed plants (n = 5, P=0.18). (c) In plots that received WCR eggs and *H. megidis,* roots from transformed plants had significantly less damage than roots from control lines (*n* = 30, *P*=0.001). Approximately one quarter of the transformed plants were found not to emit EβC. Removing these plants from the statistical analysis did significantly affect the results. Letters above bars indicate significant differences within a graph. Error bars indicate standard errors.

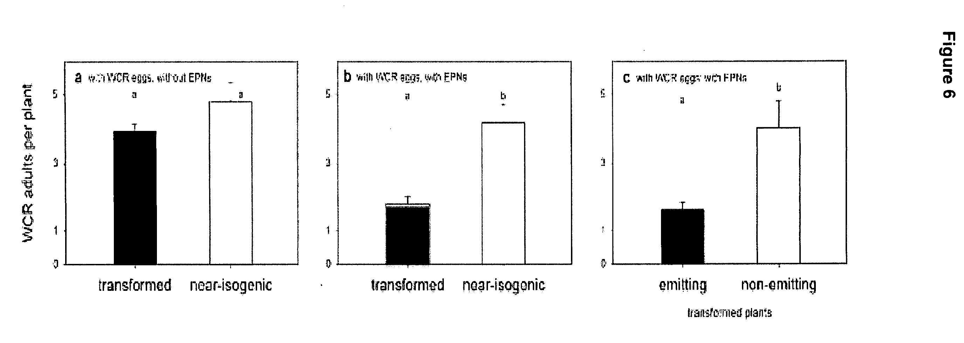

- **Figure 6**: shows that the mean number of emerging adult WCR beetles near transformed plants decreased significantly when EPN were applied. (a) Adult emergence for plants from the plots that received WCR eggs only. No significant difference was found between the transformed and non-transformed plants (n = 5, P=0.45). (b) Adult emergence for plants that received both WCR eggs and nematodes applied. A significant difference in adult emergence was found between transformed plants and control plants (n = 30, *P*<0.0001). Approximately one quarter of the transformed plants were found not to emit EβC. Removing these plants from the statistical analysis slightly reduced the average emergence near the transformed plants (black bar) and increased the difference with the plants in the control rows to 60%. (c) Significantly fewer adults emerged near EβC producing transformed plants (black bar) than near transformed plants that were not emitting EβC (cross-hatched bar, P = 0.023). Letters above bars indicate significant differences within a graph. Error bars indicate standard errors.

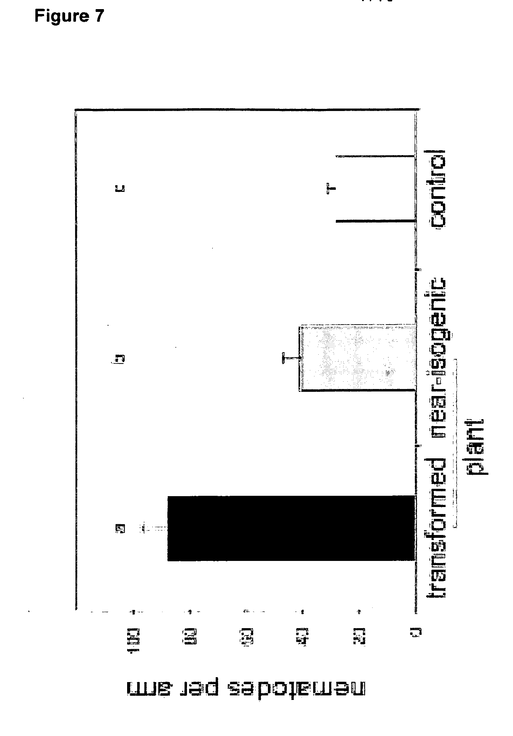

- **Figure 7**: shows that the attraction of EPN in six-arm olfactometers was higher to transformed plants than to untransformed plants. The graph depicts the average number of nematodes recovered from olfactometer arms connected to pots containing either a WCR-infested transformed plant (black bar), a WCR-infested control plant (grey bar), or only moist sand (white bar). The plants were each infested with five second instar WCR larvae. For each replicate, the total number of EPN found in the four moist sand-only control pots (white bar) were summed and divided by four. The attraction to transformed plants was significantly higher than to the control plants (n = 11, P < 0.001). Letters above bars indicate significant differences. Error bars indicate standard errors.

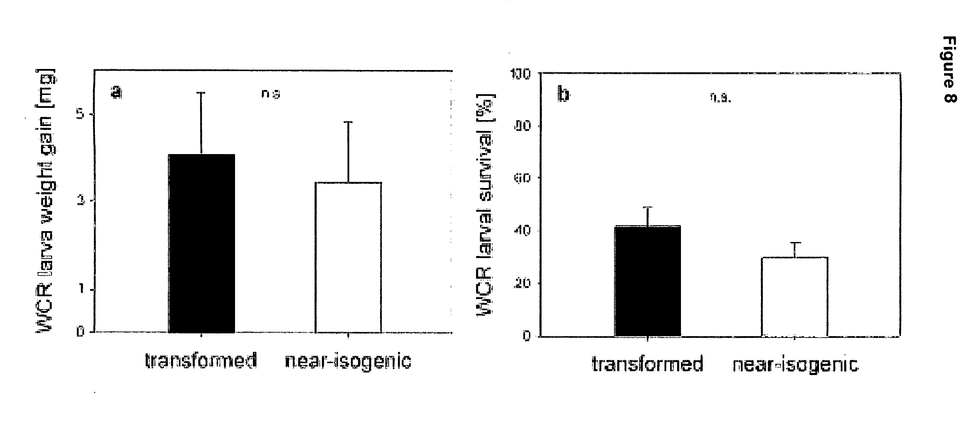

- **Figure 8**: shows that there was no direct effect of the transformation on WCR performance. (a) Average weight gain of WCR larvae fed for five days on transformed or control plants. No statistical difference was found (n = 13, P = 0.75). (b) Survival of WCR larvae after five days on transformed or non-transformed plants. No statistical difference was found (n = 13, P = 0.18). Error bars indicate standard errors.

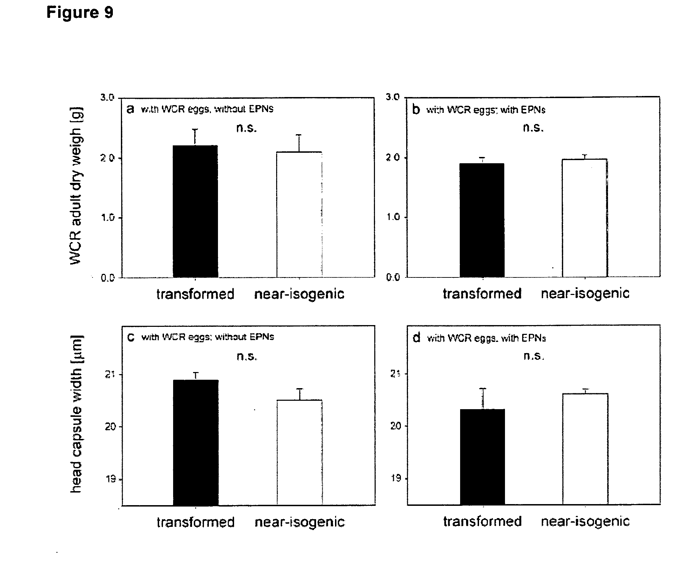

- **Figure 9**: shows the WCR adult head capsule width and weight. To evaluate if there was a difference in the quality shape size of the adult beetles that emerged near transgenic and non-transgenic plants in the two plot types that had received WCR eggs, the head capsule width and dry weight of each beetle was measured. (a) Average head capsule width of beetles that emerged in maize plots infested with WCR eggs without entomopathogenic nematodes application (P=0.57). (b) Average head capsule width of beetles that emerged in maize plots infested with WCR eggs and application of entomopathogenic nematodes (*P*=0.41). (c) Average dry weight of beetles that emerged in maize plots infested with WCR eggs without entomopathogenic nematodes application (P=0.39). (d) Average dry weight of beetles that emerged in maize plots infested with WCR eggs and application of entomopathogenic nematodes (*P*=0.94). No significant differences (n.s.) were found. Error bars indicate standard errors.

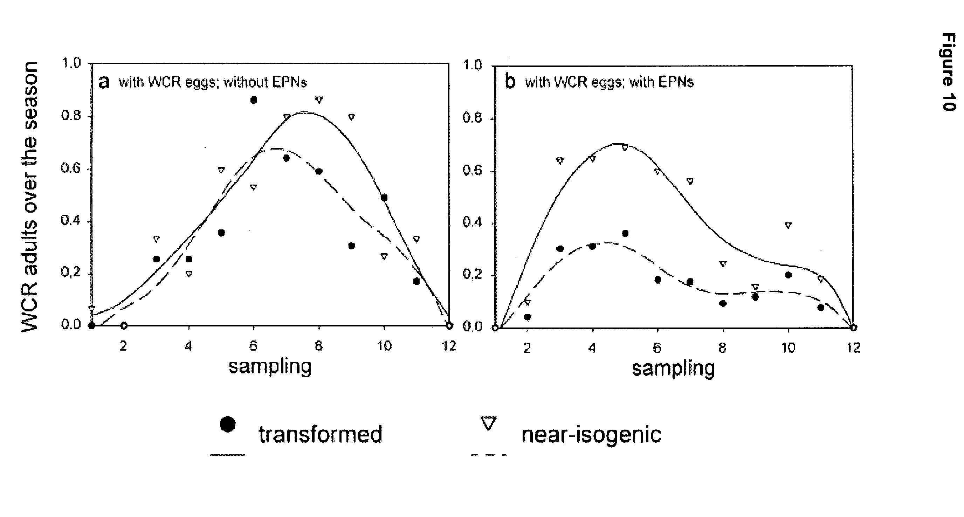

- **Figure 10**: shows the emergence of WCR adults over the collection period. WCR adults were sampled from July 17 to August 16. The number of adults collected per sampling date was plotted for the two plot types that had received WCR eggs to visualize any time effect on the emergence. (a) WCR emergence over time in maize plots infested with WCR eggs without entomopathogenic nematodes application. (b) WCR emergence over time in maize plots infested with WCR eggs and application of entomopathogenic nematodes (EPN). The late decrease of the adult emergence in plots treated with EPN may reflect an effect of multiple generations of the EPN. Once nematodes reached and infected their first hosts, a new generation of infective juveniles can be expected to emerge from this host within 10 days. A minimum of three generations must have exponentially increased the EPN population throughout the season, thereby having the most significant effect on the WCR larvae with slowest development.
- **Figure 11**: shows a dendrogramm analysis and an alignment of oregano *Ov*TPS6 (both variants described herein) with (*E*)-beta-caryophyllene synthases from other plants and with a germacrene D synthase from basil. (a) Dendrogram analysis. (b) Amino acid alignment. (c) Nucleotide alignment. The nucleotides identical to those of *Ov*TPS6-d are replaced by dots. Only the nucleotides differing from the *Ov*TPS6-d sequence are shown. (AF472361: Artemisia annua beta-caryophyllene synthase; AY640155: Cucumis sativus beta-caryophyllene synthase; AY693644: Ocimum basilicum germacrene D synthase; DQ872158: Oryza sativa (japonica cultivar-group) (E)-beta-caryophyllene (TPS3); EU259635: Zea mays cultivar Delprim (E)-beta-caryophyllene (tps23))

The following Examples serve to further illustrate the invention.
In the Examples the following materials and methods were used.

### 1. Molecular biological techniques

Unless stated otherwise in the Examples, all recombinant DNA techniques are performed according to protocols as described in Sambrook and Russell (2001), Molecular Cloning: A Laboratory Manual, CSH Press, Cold Spring Harbor, NY, USA or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, jointly published by BIOS Scientific Publications Ltd (UK) and Blackwell Scientific Publications (UK).

### 2. Plant and insect material

Oregano (*Origanum vulgare* L.) plants were propagated from stem cuttings and grown in a greenhouse with addtional lighting by sodium lights (16 h day at 20-22 °C and 18-20 °C at night), 30 - 55 % humidity. Three clonal lines were used propagated from the cultivar d06-01 (d2, d5, d8) and four lines from the cultivar f02-04 (f2 - f5). Glandular trichome secretory cell clusters were extracted from the surface of young leaves and subsequently used for RNA extraction in order to generate cDNA libraries.
Seeds of the maize hybrid line Hill is available at the Maize Genetics Cooperation Stock Center, USDA/ARS & Crop Sciences/UIUC, S-123 Turner Hall, Urbana, IL 61801-4730. The plants were grown in commercially available potting soil in a climate-controlled chamber with a 16 h photoperiod, 1 mmol (m²)⁻¹ s⁻¹ of photosynthetically-active radiation, a temperature cycle of 22 °C/18 °C (day/night) and 65% relative humidity. Twelve to fifteen day old-plants (20-30 cm high, 4-5 expanded leaves) were used in all experiments.
Larvae from *Diabrotica virgifera virgifera* LeConte (Western corn rootworm, WCR) were obtained from CABI BioSience (Delémont, Switzerland). The larvae are reared in pots with maize plants growing in commercially available potting soil in a climate-controlled chamber with a 16 h photoperiod, 1 mmol (m²)⁻¹ s⁻¹ of photosynthetically-active radiation, a temperature cycle of 22 °C/18 °C (day/night) and 65% relative humidity.
The nematode *Heterorhabditis megides* (Rhabditida : Heterorhabditidae) were obtained from Becker Underwood Ltd, UK, or Andermatt Biocontrol AG (Grossdietwil, Switzerland). The nematode preparation (Meginem) was dissolved in water and applied to the soil according to the manufacturer's instructions.

### 3. cDNA library construction

Peltate glandular trichomes were isolated from young leaves using a method modified from Gershenzon et al. (1992). In brief, approx. 7 g of young, not fully expanded leaves of the cultivars d06-01 or f02-04 were harvested, soaked in ice cold, distilled water containing 0.05 % Tween20 for 2h. The water was then decanted and the leaves were washed twice with ice-cold, distilled water, and abraded using a cell disrupter (Bead Beater, Biospec Products, Bartlesville, USA). The chamber was filled with the plant material, 65 ml of glass beads (0.5 - 1.0 mm diameter), XAD-4 resin (1 g/g plant material), and ice-cold extraction buffer (25 mM HEPES pH 7.3, 12 mM KCI, 5 mM MgCl₂, 0.5 mM K₂HPO₄, 0.1 mM Na₄P₂O₇ 5 mM DTT, 2.4 g I⁻¹ sucrose, 26.4 g I⁻¹ D-sorbitol, 6 g I⁻¹ methyl cellulose, and 10 g I⁻¹ polyvinylpyrrolidone (PVP; Mr 40,000)) to full volume. Glands were abraded from the leaves with three pulses of 1 min at medium speed, with a 1-min rest between pulses. Following abrasion, the glands were separated from leaf material, glass beads, and XAD-4 resin by passing the supernatant of the chamber through a 500-µm mesh cloth. The residual plant material and beads were rinsed twice with 10 ml ice-cold isolation buffer (extraction buffer without methylcellulose) and passed through the 500-µm mesh. The combined 500-µm filtrates were then consecutively filtered through membranes with decreasing mesh size (350-µm, 200-µm, and 100-µm). Finally, clusters of secretory cells (approx. 60 µm in diameter) were collected by passing the 100-µm filtrate through a 20-µm mesh. An aliquot of the isolated cell clusters were checked for integrity and purity with a light-optical microscope before being transferred to a 1.5 ml reaction tube and frozen in liquid nitrogen prior to RNA extraction.
RNA from isolated glandular trichomes was prepared and further purified by using Trizol reagent (Invitrogen, Clasbad, USA) and the RNeasy Plant Mini kit (Qiagen, Hilden, Germany) following the manufacturer's instructions. A directional cDNA library was constructed from 1µg total RNA using the Creator Smart cDNA library construction kit (BD Bioscience Clontech, Mountian View, CA, USA) following the manufacturer's protocol, except using Superscript III reverse transcriptase (Invitrogen, Carlsbad, USA) to perform the cDNA fist strand synthesis.
Large scale sequencing of 2,364 clones from a cDNA library of *O*. *vulgare* cultivar f02-04 was performed at the Purdue University Genomics Core Facility (Purdue University West Lafayette, Indiana, USA). Sequences were assembled with the SeqMan program (V5.05, DNAStar, Madison, USA) and the resulting contigs were compared by BLAST search via the NCBI sequence database (http://www.ncbi.nlm.nih.gov/BLAST/). Sequences with similarities to terpene synthases genes from other plants were used to design primers for the isolation of full length cDNAs clones by RACE-PCR. For this procedure, the primers were used with the cDNA libraries of the cultivars d06-01 and f02-04 and the BD SMART RACE cDNA Amplification Kit (BD Bioscience Clontech, Mountian View, CA, USA). The conditions for the PCR reaction were: 0.8 µl Adv. Taq DNA Polymerase Mix (5 U/µl), 5 µl 10 x Adv. Taq PCR-buffer, 1 µldNTPs (10 mM each), 5 µl universal primer mix primer mix and 1 µl gene-specific primer (10 pmol/µl) 0.5 - 1 µl cDNA and PCR grade water was added to final volume of 50 µl. The PCR was conducted with an initial denaturation at 94 °C for 2 min, 30-35 cycles of denaturation at 94 °C for 30 s, annealing at 52 °C to 57 °C for 30 s and extension at 68 °C for 60 s to 150 s, and a final step at 70 °C for 5 min. PCR fragments were analyzed by cloning into pCR4-TOPO vector (TOPO TA cloning kit for sequencing, Invitrogen, Carlsbad, USA) and subsequently sequenced. RACE-PCR was repeated several times to verify the correct 5' and 3' ends of the cDNAs. cDNA fragments from at least two independent RACE-PCR reactions were fully sequenced to prevent sequence errors.

### 4. Functional expression of terpene synthase

Signal peptide predictions were performed with the ChloroP and TargetP programs (Emanuelsson et al. 2007). Expression constructs from complete open reading frames of the putative terpene synthases *Ov*TPS6 were cloned into the bacterial expression vector pH9GW. This vector is a derivative of the pET-T7 (28a) vector (Novagen, Madison, WI, USA) containing a 9xHis-tag followed by a gateway attR cassette (Yu and Liu 2006). Gene amplification was performed with primers (see Table 1 in supplemental material) using the Advantage Taq Polymerase (BD Bioscience Clontech, Mountian View, CA, USA) as directed by the manufacturer. The amplification products were cloned into pCR4-TOPO vector (Invitrogen, Carlsbad, USA). Expression constructs were created with the gateway technology (Invitrogen, Carlsbad, USA). Validated sequences were subcloned from the pCR4-TOPO vector into the pDONR207 vector with BP Clonase II (Invitrogen, Carlsbad, USA). Subsequently, constructs were cloned into the pH9GW expression vector LR Clonase II according to the manufacturer's instructions. The expression constructs were verified by sequencing and transformed into the BL21 (DE3) strain (Invitrogen, Carlsbad, USA). For expression, a starter culture (5 ml Luria-Bertani (LB) medium with 100 µg ml⁻¹ kanamycin) was grown over night at 37°C. The expression culture (50 µl starter culture in 125 ml LB medium with 100 µg ml⁻¹ of kanamycin) were grown in auto-induction terrific broth (TB) media (Overnight express instant TB medium, Novagen, Madison, WI, USA). Cells were harvested by centrifugation for 5 min at 5,500 x *g.* Pellets were resuspended in 3 ml extraction buffer (50 Mm MOPSO buffer at pH 7.0, 10 % (v/v) glycerol, 5 mM DTT, 5 mM Na-ascorbate, 0.5 mM PMSF) and disrupted by sonification (Bandelin sonopuls HD 2070, Bandelin electronics, Berlin, Germany) for 3 min, cycle 2, power 65%. Cell fragments were removed by centrifugation at 16,100 × g for 30 min at 4 °C. The supernatant contained the expressed proteins. Buffer exchange into assay buffer (20 mM MOPSO buffer at pH 7.0, 10 % (v/v) glycerol, 1 mM DTT) was performed with 10DG columns (BioRad, Hercules, CA, USA). Enzyme activities were assayed in 1-ml volumes containing 200 µl of the extracted enzyme. Assays contained 10 µM GPP or FPP (both Echelon, Salt Lake City, USA). Metal ions were added at the concentrations 0.1 mM NaWo₄, 0.05 mM NaF and 1 mM MgCl₂ or 0.5 mM MnCl₂. Terpenoid products were collected by a device for solid phase micro extraction (SPME) equiped with a polydimethylsiloxane coated fiber. The fiber was exposed to the head space above the assay mixture at 30 °C in a water bath for 30 to 60 min.

### 6. Gas chromatography

Products of terpene synthase assays were identified by gas chromatography (Agilent Hewlett-Packard 6890, Agilent Technologies, Santa Clara, CA, USA) coupled to a Network Mass Selective Detector (MS) (Agilent Hewlett-Packard 5973, Agilent Technologies) or a Flame Ionization Detector (FID). For analyses, 2 µl of pentane or ethylacetate:pentane (2:1) extracts were injected at 230 °C. Alternatively, the solid phase micro extraction (SPME)-fiber was introduced into the injector at 180 °C. The terpenes were separated on a DB5-MS column (30 m length, 0.25 mm inner diameter and 0.25 µm film by J&W Scientific; GC-program 40°C for 2 min, first ramp 5 °C min⁻¹ to 175 °C, second ramp 90 °C min⁻¹ to 250 °C, final 3 min hold). GC-MS carrier gas: helium at 1 ml min⁻¹; GC-FID carrier gas: hydrogen at 2 ml min⁻¹. All terpene products were identified by using Agilent Technologies software with the Wiley 275.L and NIST 98.1 MS libraries, as well as by comparison of mass spectra and retention times with those of authentic standards (Sigma-Aldrich Chemicals, Steinheim, Germany).

### 8. Sequence and phylogenetic analyses

The amino acid alignment was conducted by use of ClustalX (Protein weight matrix: Gonnet series; gap opening : 10.00; gap extension: 0.20; delay divergent sequences: 30 %) (Thompson et al. 1997). The resulting tree was visualized with Treecon 1.3b (Bootstrap: 1000) (Van de Peer and De Wachter 1994).

### 9. Transformation

The general procedure for the transformation of the maize line Hill has been described by Frame et al., 2002. The Ti-plasmid pTF101.1 was kindly provided by the Plant Transformation Facility, lowa State University, Ames, lowa, USA. Plasmid pTF101.1 derived from pTF102. It lacked the β-glucuronidase-reporter gene and instead expressed the phosphoinothricin acetyl transferase from *Streptomyces hygroscopicus* gene via a double Cauliflower Mosaic Virus 35S promoter. Inserted in this vector was the (*E*)-β- caryophyllene synthase *tps6* from *Origanum vulgare* under control of a maize ubiquitin promoter (Torney et al., 2004). To facilitate cloning, a EcoRI restriction site within the nucleotide sequence was destroyed by site-directed mutagenesis which resulted in the alteration of amino acid 423 from cysteine to threonine. The structural gene was followed by a terminator from nopaline synthase of the *Agrobacterium tumefaciens* Ti plasmid. The transgenic lines 202 L1, 202 L2 and 202 L5 were regenerated from three independent transgenic calli generated in two independent transformation experiments. Selfed T1 generations of the three transformed lines were used in the experiments. As non-transformed controls, the selfed F1 progeny of Hill as well as the T1 progeny of a transgenic line with no expression of the EβC synthase gene, 197 L1, were used.

### 10. Field experiment

All experimental plots were hand-planted on May 22 (2007) at the Bradford Research and Extension Center, Columbia, Missouri, USA. Each plot had rows of eight plants of a particular line with 43 cm spacing between plants and 76 cm spacing between rows. For the main experiment there was a total of thirty plots. Each plot had a row of control plants and a row of a transformed line (Fig.4 a). There were ten plots for each of the three transformed lines and all plots received WCR eggs as well as nematodes. In addition, we planted ten control plots that did not receive nematodes. Half of the control plots were infested with WCR. Each control plot had four rows: three rows with each eight plants of a particular transformed line and one row with eight control plants (Fig. 4b, c). Plots were randomly distributed in the field, with each plot surrounded by two rows of maize plants of the variety Pioneer 3394, serving as a buffer between plots. The above plantings and infestation procedures resulted in three types of plots (Fig. 4): 1) thirty principal experimental plots with WCR infestation and nematode release, 2) five plots without WCR and without nematodes, and 3) five plots that had only WCR and no nematodes.
On June 4, all plots except half (five) of the control plots were infested with WCR eggs. Eggs were mixed into a solution of water and 0.15% agar and each plant received about 400 viable eggs in two 10 cm deep holes at a distance of 5 cm from the plant (∼800 eggs per plant). On June 28, about 600,000 *H. megidis* (Becker Underwood Ltd, UK) were applied in between the two rows of the thirty experimental plots. Nematodes were mixed in 0.5 I of water and the solution was poured into a 2 cm deep trench that was dug between the rows. The number of EPNs that was used is equivalent to 250,000 EPN per square meter. No nematodes were applied to the ten control plots. Plans had been made to irrigate before and after infection with EPN, but 5.9 cm of rain fell starting the day before infection, extending 3 d after infection, including 3.76 cm on the day of infection. On July 11 root damage was assessed by digging out half of the plants in each plot (four plants per row). Plants were immediately and carefully removed from the field in order to avoid nematode and WCR contamination of the neighbouring plants. The root systems were washed and damage from WCR larval feeding was rated using a 0-3 root scale34.
The 400 remaining plants stayed in the field and emergence cages (78 x 36 cm) modified after Pierce and Gray (2007) were placed over each plant on July 11. Traps were checked three times per week during the peak of WCR emergence and twice per week during periods when few adults were emerging. All beetles collected were placed in individually labelled scintillation vials (J & H Berge, Inc., Plainfield, NJ USA) containing 95% ethanol and brought to laboratory for processing. Firstly, beetles were counted and sexed by emergence date. Secondly, the head capsule width of each beetle was measured using an ocular micrometer (10x/21, Wild Co., Heerbrugg, Switzerland) mounted on a microscope (M3Z, Wild Co., Heerbrugg, Switzerland). Upon these two measurements, beetles were placed in a desiccating oven (Thelco Model 16, GCA/Precision Scientific Co., Chicago, IL) at 60 oC for 48 h. The dried beetles were then placed on an analytical scale (Model AB135-S FACT, Mettler, Columbus, OH), and total dry weight was recorded.
Finally, corn ears were hand-harvested during the third week of September to assess yield. Ears were dried to 15.5% moisture prior to shelling and cleaning, after which the dry weight of the combined seeds of each plant was determined. PROC MIXED of the statistical package SAS (SAS Institute 1990) was used for data analyses. A separate analysis was done for plant damage, adult emergence, beetle dry weight, and head capsule width. Data were analysed as a randomized complete block with treatments arranged in a 5 × 2 factorial (5 maize genotypes × 2 experiments) as outlined in Steel et al. (1997). The reason for this was that half of the blocks contained a randomization of the main experimental plots (WCR plus nematodes) and the control plots with WCR and the other half contained a randomization of the main experimental plots and control plots without WCR. First, a comparison among the three transformed lines revealed no significant differences within the data sets. Therefore, these lines were pooled for further analyses. A separate comparison between the two isolines sources of the original non-transformed line also revealed no significant differences in any of the above factors and they were also pooled for subsequent analyses. Finally, selected contrasts were made between specific treatments within each type of plots following the methods outlined in Littell et al. (1998). Difference in WCR adult emergence between transformed plants producing EβC or not was analysed with SPSS 14.0. As homogeneity of variance test failed, means were compared with the nonparametric Mann-Whitney test.

### 11. Screening for the presence of native EPN

Three soil samples along the application trench were taken two days prior to (June 26) and again seven days after the nematode *H. megidis* application (July 5). Each sample contained approximately 200 g soil from an area of 5 cm² and was kept at 4°C until it was used for the next step. Detection of nematodes was done with the *Galleria mellonella* baiting method. Soil was homogenized by hand and each soil sample was placed in a 350 ml glass jar (7cm diam., 12cm deep) with three final instar larvae of G. *mellonella.* After the larvae had been added, the boxes were turned round and stored at 25°C in darkness. Five days later, larvae were examined for nematode infection. Larvae with the typical red colour, indicative of the Heterorhabiditidae bacterial symbiont introduced into the host by EPN, were recorded as infected. As other families of EPN could also have infected the larvae, the remaining cadavers were dissected and checked for EPN presence/absence.

### 12. EβC emission screens

Leaf clippings (∼25 cm²) were sampled from all plants the day before harvesting plants used for root damage rating (on July 10). These clippings were frozen in the field by placing them in liquid nitrogen and subsequently stored in a -80°C freezer. Before chemical analyses, individual leaf samples were ground in liquid nitrogen and about 0.3 g of leaf powder was placed in a 20 ml glass vials. Using a multi-purpose-sampler (MPS2, Gerstel GmbH & Co.KG, Germany) a 100 µm polydimethylsiloxane solid-phase microextraction (SPME) fibre (Supelco, Buchs, Switzerland) was inserted in the vial through a septum in the lid and exposed for 60 min at 40°C. The compounds adsorbed on the fibre were analysed with an Agilent 6890 Series (G1530A) gas chromatograph coupled to a quadrupole-type mass-selective detector (Agilent 5973; transfer line 230°C, source 230°C, ionization potential 70 eV). The fibre was inserted into the injector port (230°C, 50 mm), desorbed for 3 min and chromatographed on a DB5-MS column (30 m, 0.25 mm internal diameter, 0.25 µm film thickness; J &W Scientific). Helium at a constant pressure of 18.55 lb in-2 (127.9 kPa) was used for carrier gas flow. After fibre insertion, the column temperature was maintained at 60°C for 1 min and then increased to 270°C at 10°C min⁻¹ ended by a final stage of 5 min at 270°C. Chromatograms were analysed using ChemStation D.00.00.38.

### 13. Olfactometer assays

Olfactometer assays were conducted as described by Rasmann et al. (2005). Olfactometers consist of six glass pots connected via glass tubes to a central pot. Each system of seven pots was filled with 10% moist sand (Migros, Switzerland). A twelve day old transformed plant (line 202 L2) that had been grown in a climate chamber (Weiss Technik, Switzerland, 16:8 light/dark hours, 25:15°C day/night temperature and 60% humidity) in a mix of potted soil and sand was transplanted in sand in one of the outer pots (5 cm diam., 11 cm deep). A similar non-transformed plant (line 197 L1) was placed in another sand-filled pot. The four remaining pots only contained sand. The pots with the plants each received five second instar WCR larvae that were weighed as a group just before. Two days after WCR infestation, the posts were connected to the olfactometer central pot (8 cm diam., 11 cm deep) using a glass connector (8 cm long; 24/29 male connector on both sides, all glassware from VQT-Verre Quartz Technique SA, Switzerland) and a Teflon connector (24/29 female to 29/31 male) containing an ultra fine mesh metal screen (2300 mesh; Small Parts Inc., FL, USA), which prevented the nematodes from entering the odour source pots. One day later, about 2,000 *H. megidis* nematodes were released in the central pot and the following day the olfactometers were disassembled. Sand contained in the glass and Teflon connectors was separately placed on cotton filters (19 cm diam., Hoeschele GmbH, Switzerland). Filters and sand were placed on a Bearmann extractor and recovered nematodes were counted the following day.
To evaluate the performance of the WCR larvae on the plants, the larvae were left in the pots for two additional days. Then they were recovered from the pots by passing sand through a 0.6 mm sieve (Eijkelkamp, The Netherlands). Survival was determined and recovered WCR larvae were weight on a micro-scale (Mettler-Toledo GmbH, Switzerland).
The nematodes' choices among the arms of the olfactometers were examined with a log-linear model. The entity computing a repetition in the statistical analysis corresponds to the response of a group of nematodes released, which was shown to follow a multinomial distribution. As the data did not conform to simple variance assumptions implied in using the multinomial distribution, we used quasilikelihood functions to compensate for the overdispersion of nematodes within the olfactometer. WCR larvae performances differences were tested with SPSS 14.0. As normality and homogeneity of variance tests passed, survival and weigh means were compared with a t-test.

### Example 1: Isolation of a terpene synthase gene from Origanum vulgare

In order to identify the genes responsible for terpene formation in *O*. *vulgare,* cDNA libraries were generated. Glandular trichome cell clusters of the two cultivars d06-01 and f02-04 were isolated for RNA extraction to enrich for genes related to terpene biosynthesis. Sequencing of 2,364 random clones from a cDNA library of line f02-04 resulted in 69 ESTs that displayed sequence similarity to those of terpene synthases from other plants. These EST fragments were extended by several rounds of RACE-PCR to yield six individual full length cDNA clones which were designated putative terpene synthases *Ovtps*1 through 6. *Ovtps*7 was not represented in the screened cDNA library but was found by PCR with primers for *Ovtps*1. CELI-PCR (Ren et al. 2005) was used to confirm *Ovtps5*. Terpene synthase genes were isolated also from cultivar d06-01 cDNA.

### Example 2: Sequence comparison of the isolated terpene synthases and identification of the (E)-β-caryophyllene synthases (Ovtps6)

All amino acid sequences predicted from the gene's open reading frames showed high overall sequence identity to terpene synthases as well as characteristic sequence motives common to terpene synthases, notably the double arginine motif (RR(x8)W) in the N-terminal domain which is common for monoterpene synthases (Pechous and Whitaker 2004). A highly conserved aspartate-rich motif (DDxxD) which is involved in binding the bivalent metal ion cofactor (Bohlmann et al. 1999) was found in the C-terminal domain of six protein sequences. Only *OvTPS*4 displays a DDxxE motif similar to the 5-epi-aristolochene synthase (CAA06614) from *Capsicum annuum (Zavala-Paramo et al. 2000).*
Signal peptides were predicted for *Ov*TPS1 (35 aa), *Ov*TPS2 (24 aa) and *Ov*TPS7 (32 aa). The plastid was assigned as target organelle which is considered as location of monoterpene biosynthesis (Turner et al. 1999). *OvtTPS3* through *OvtTPS6* did not display signal peptides.
Sequence comparisons of *O*. *vulgare* terpene synthase genes *Ovtps1, Ovtps2, Ovtps5* and *Ovtps7* to known plant terpene synthase genes resulted in an approx. 60% amino sequence identity to monoterpene synthases of other Lamiaceae, including β-myrcene synthase (AY693649) and fenchol synthase (AY693648) of *Ocimum basilicum* (lijima et al., 2004), sabinene synthase of *Salvia officinalis* (AF051901) (Wise et al. 1998), linalool synthase of *Mentha citrata* (AY083653), bornyl synthase of *Rosmarinus officinalis* (ABP01684) and linalool synthase of *Perilla frutescens var. crispa* (AF444798). *Ovtps5* shares high sequence identity with *Ovtps2* (74.7% and 76.7% sequence identity on the amino acid level for culivars d06-01 and f02-04, respectively). The major difference between both amino acid sequences is the missing signal peptide in *Ovtps5.*
The terpene synthases *OvTPS3, OvTPS4* and *OvTPS6* showed approx. 60% amino acid identity to sesquiterpene synthases of other Lamiaceae including (-)-germacrene D synthase (AY693644) and selinene synthase (AY693643) from *Ocimum basilicum* (lijima et al., 2004).
*Ovtps6* showed relatively low sequence identity to other (*E*)-β-caryophyllene synthases from *Artemisia annua* and *Cucumis sativus* with only 41.2 to 44.0% identity (Figure 1).
Expression of *OvTPS6* resulted in an enzyme producing (*E*)-β-caryophyllene and α-humulene from farnesyl diphosphate (FPP) (Figure 2).

### Example 3: Transformation of maize with the (E)-β-caryophyllene synthase gene Ovtps6 from oregano

The maize variety Hill was transformed with the (*E*)-β-caryophyllene synthase gene *Ov*tps6 from *Origanum vulgare* under the control of a maize ubiquitin promoter (Fig. 3a). The transformation resulted in plants that produced EβC constitutively (Fig. 3b), supporting the observation that loss of EβC production in most American maize lines is due to the loss of (*E*)-β-caryophyllene-synthase activity (Kölner et al., 2008). Three independently transformed lines were selected with EβC concentrations in their roots that were either similar (line 201 L1) or six-fold higher (lines 202 L2, 202 L5) than what is typically present in our model European maize line Delprim (Rasmann et al., 2005) (Figure 3c). Since sesquiterpene hydrocarbons like EβC appear to be emitted from maize by passive diffusion (Köllner et al., 2004), the roots of transgenic maize containing higher concentrations of (*E*)-β- caryophyllene can be expected to release proportionally higher levels of this compound. For the following field experiments, selfed T1 generations of the three transformed lines were tested. As control plants we used F1 progeny of the selfed non-transformed Hill, as well as the T1 progeny of a transgenic line (197 L1) with no expression of the EβC synthase gene, thus providing the highest genetic similarity to the transformed plants.
Since the transformed lines segregated 3:1 for the transgene, a quarter of the progeny did not produce EβC. Therefore, all transformed plants used in field experiments were tested for EBC production in order to accurately evaluate the results.

### Example 4: Field experiments comparing lines transformed with the (E)-β-caryophyllene synthase gene Ovtps6 from oregano and control lines

Field tests were conducted to determine whether the EβC emission of the transgenic plants enhanced the ability of *H*. *megidis* to find and kill WCR under realistic conditions and so reduce plant damage. In a 20 by 70 meter maize field thirty experimental plots were planted, each consisting of one row with eight plants of a particular transformed line alongside a row with eight plants of a control line (Fig. 4a). Two weeks after planting, all plots were infested with WCR eggs and another three weeks later 600,000 *H. megidis* were applied between the two rows of each plot. In addition to these two-row experimental plots, we planted ten control plots with each four rows: three rows with each eight plants of a particular transformed line and one row with eight control plants (Fig, 4b, c). Five of the control plots also 114 received WCR eggs, but no nematodes were applied to any of the plots.
The effectiveness of the EPN in controlling WCR was evaluated by measuring root damage and emergence of WCR beetles. Root damage (0-3 node-injury scale (Oleson et al., 2005)) was assessed two weeks after nematode application by digging out four of the eight plants in each row. Emergence of WCR adults was monitored throughout the summer by placing emergence cages over each plant that remained in the experimental plots. Damage to roots in experimental plots without WCR was minimal, but considerable root damage was found in plots that had received only WCR eggs (Fig. 5a, b). Without *H. megidis,* there was no difference in WCR-caused root damage between transformed plants and the control plants. In accordance, the number of emerging WCR beetles (approximately four per plant) was the same for transformed and control plants (Fig. 6a). There was no WCR emergence in the plots that were not infested with WCR eggs, but eight *Diabrotica barberi* (northern corn rootworm) beetles were captured in the emergence cages within these plots.
In plots in which *H. megidis* nematodes were released, the transformed plants received significantly less root damage than the controls (Fig. 5c), as would be expected from the attractiveness of EβC release. In accordance, adult WCR emergence per plant was reduced to less than half with transformed plants as compared to the control plants (Fig. 6b).
Because all seeds used for the experiments were produced by selfed plants the transformed lines segregated 3:1 for the transgene and only a quarter of the plants should produce EβC. This provided an additional built-in fully blind control experiment. The EβC emission was checked from clippings of leaves obtained 137 from all plants the day before harvesting the plants that were used for the evaluation of root damage. The results for the transformed plants were corrected by discarding the plants that did not produce EβC (26.8%) from the data analyses. This correction had no effect on the average for root damage, but the average adult emergence near EβC emitting plants was reduced by another 5%, making it 60% less than near non transformed plants (Fig. 6b). Moreover, there was a striking difference in the average number of adults that emerged near EβC-producing plants compared to the quarter of their sister plants that did not produce EβC in the same rows (1.79 vs. 3.80 beetles per plant, Fig. 6c).
Of each emerging beetle, the head capsule width and dry weight was determined, but no differences for these parameters were found among treatments (Fig. 9). Emergence of beetles started in mid July and lasted until mid August (Fig. 10). Comparison of the emergence over time between plots with and without *H. megidis* shows that the nematode mostly suppressed WCR emergence at the end of the season (Fig. 10). This late effect is likely due to increasing numbers of *H. megidis,* as a new generation of infective juveniles will emerge within two weeks after infection, allowing the nematode to multiply over several generations during the season. The data are consistent with the hypothesis that the transformation with (*E*)-β-caryophyllene resulted in increased attraction of applied *H. megidis* and thereby enhanced WCR mortality and root protection. However, we considered and tested two additional explanations for increased root protection: that EβC attracts native nematodes which protected the plants or that the EβC-releasing transformants had a direct negative effect on the performance of WCR larvae.

### Example 5: Eliminating alternative explanations: Evaluation of the presence of naturally occurring entomopathogenic nematode populations in the experimental field

In order to evaluate the presence of naturally occurring entomopathogenic nematode populations in the experimental field, soil samples were collected from the plots before and after the application of *H. megidis* (see Methods). Waxmoth larvae (*Galleria mellonella* L., Lepidoptera: Galleridae) were incubated in these soil samples and nematode infection was determined five days later. In soil collected before H. *megidis* release, 4.9 % of the larvae were infected, whereas in soil from after release 59.2% were infected. These results imply that there was indeed an effect of native EPN on WCR infection rates during the experiments. Interestingly, the tendency of reduced beetle emergence near transgenic plants in the control plots was only apparent at the end of the season (Fig. 10a), when the native EPN population can be expected to have had built up its numbers.

### Example 6: Eliminating alternative explanations: Test for a possible direct effect of the (E)-β-caryophyllene synthase transformation on WCR performance

To test for a possible direct effect of the (*E*)-β-caryophyllene synthase transformation on WCR performance, as well as to confirm enhanced attractiveness of transformed plants to *H. megidis,* we conducted an additional laboratory experiment with the use of six-arm belowground olfactometers (Rasmann et al., 2005). These consisted of six glass pots that can be connected via glass tubes to a central pot with the whole system filled with moist sand. A twelve-day old transformed plant was transplanted in one of the six outer pots, a similar non-transformed plant was placed in the opposite pot, and the four remaining pots contained only sand. The pots with the plants each received five second instar WCR larvae that were weighed as a group just before. Three days after introducing the WCR larvae, about 2,000 *H. megidis* nematodes were released in the central pot. The nematodes could crawl into the arms, but they were prevented from entering the outer pots by an ultra-fine metal screen at the end of each arm (see Methods and Rasmann et al., 2005 for details). The day after their release, the nematodes in each of the olfactometer arms were counted. To evaluate the performance of the WCR larvae on the plants, the larvae were left in the pots for an additional two days. Then they were recovered from the pots and their survival and weight were determined. Consistent with the reported (Rasmann et al., 2005; Köllner et al., 2008) attractiveness of EβC, significantly more nematodes were recovered from the olfactometer arms connected to the pot with the WCR-infested transformed plants than from those connected to the pots with the infested non-transformed plants (Fig. 7). The transformation had no direct effect on the performance of WCR larvae. Average weight increase and survival were the same for larvae that had fed for five days on transformed plants as compared to larvae that had fed the same period of time on non-transformed plants (Fig. 8). These results support the conclusion that the improved control of WCR near transformed plants in the field experiments was due to the enhanced attractiveness of the roots to EPN and not to a direct negative effect on the WCR larvae.

### Cited literature

Bohlmann J., Phillips M., Ramachandiran V., Katoh S., Croteau R. (1999) cDNA cloning, characterization, and functional expression of four new monoterpene synthase members of the Tpsd gene family from grand fir (Abies grandis). Archives of Biochemistry and Biophysics 368: 232-243

Cai Y., Jia J.W., Crock J., Lin Z.X., Chen X.Y., Croteau R. (2002) A cDNA clone for β-caryophyllene synthase from Artemisia annua. Phytochemistry 61, 523-529

Degen T., Dillmann C., Marion-Poll F., Turlings T.C.J. (2004) High genetic variability of herbivore-induced volatile emission within a broad range of maize inbred lines. Plant Physiology. 135(4): 1928-1938

Emanuelsson O., Brunak S., von Heijne G., Nielsen H. (2007) Locating proteins in the cell using TargetP, SignalP and related tools. Nat. Protocols 2: 953-971

Frame B.R., Shou H., Chikwamba R.K., Zhang Z., Xiang C., Fonger T.M., Pegg S.E., Li B., Nettleton D.S., Pei D., Wang K. (2002) Agrobacterium tumefaciens-mediated transformation of maize embryos using a standard binary vector system. Plant Physiology 129: 13-22.

Gershenzon J., Kreis W. (1999) Biosynthesis of monoterpenes, sesquiterpenes, diterpenes, sterols, cardiac glycosides and steroid saponins. In Biochemistry of Plant Secondary Metabolism, Annual Plant Reviews, Vol. 2., Wink M (ed) pp 222-229. Sheffield: Sheffield Academic Press.

Gershenzon J., Mccaskill D., Rajaonarivony J.I.M., Mihaliak C., Karp F., Croteau R. (1992) Isolation of Secretory-Cells from Plant Glandular Trichomes and Their Use in Biosynthetic-Studies of Monoterpenes and Other Gland Products. Analytical Biochemistry 200: 130-138

lijima Y., Davidovich-Rikanati R., Fridman E., Gang D.R., Bar E., Lewinsohn E., Pichersky E. (2004) The biochemical and molecular basis for the divergent patterns in the biosynthesis of terpenes and phenylpropenes in the peltate glands of three cultivars of basil. Plant Physiology 136: 3724-3736

Jackson J.J. (1996) Field performance of entomopathogenic nematodes for suppression of western corn rootworm (Coleoptera: Chrysomelidae). Journal of Economic Entomology 89: 366-372

Jackson J. J. & Hesler L. S. (1995) Placement and application rate of the nematode Steinemema carpocapsae (Rhabditida: Steinernematidae) for suppression of the western corn rootworm (Coleoptera: Chrysomelidae). Journal of the Kansas Entomological Society 68: 461-467

Journey A. M. & Ostlie K. R. (2000) Biological control of the western corn rootworm (Coleoptera : Chrysomelidae) using the entomopathogenic nematode, Steinernema carpocapsae. Environmental Entomology 29: 822-831

Köllner T. G. et al. (2008) A Maize (E)-{beta}-caryophyllene synthase implicated in indirect defense responses against herbivores is not expressed in most American maize varieties. Plant Cell 20: 482-494

Köllner, T. G., Schnee, C., Gershenzon, J. & Degenhardt, J. (2004) The sesquiterpene hydrocarbons of maize (Zea mays) form five groups with distinct developmental and organ-specific distributions. Phytochemistry 65, 1895-1902

Littell R. C., Henry P. R., Ammerman C. B. (1998) Statistical analysis of repeated measures data using SAS procedures. Journal of Animal Science 76: 1216-1231

Nishimatsu T. & Jackson J. J. (1998) Interaction of insecticides, entomopathogenic nematodes, and larvae of the Western Corn Rootworm (Coleoptera: Crysomelidae). Journal of Economic Entomology 91: 410-418

Oleson J. D., Park Y. L., Nowatzki T. M. & Tollefson J. J. (2005) Node-injury scale to evaluate root injury by corn rootworms (Coleoptera : Chrysomelidae). Journal of Economic Entomology 98: 1-8

Pechous S.W., Whitaker B.D. (2004) Cloning and functional expression of an (E,E)-alpha-farnesene synthase cDNA from peel tissue of apple fruit. Planta 219: 84-94

Pierce C. M. F. & Gray M. E. (2007) Population Dynamics of a Western Corn rootworm (Coleoptera: Chrysomelidae) Variant in East Central Illinois Commercial Maize and Soybean Fields. Journal of Economic Entomology 100: 1104-1115

Rasman S., Köllner T.G., Degenhardt J., Hiltpold I., Töpfer S., Kuhlmann U., Gershenzon J., Turlings T.C.J. (2005) Recruitment of entomopathogenic nematodes by insect-damaged maize roots. Nature 434: 732-737

Ren M.Z., Chen Q.J., Li L., Zhang R., Guo S.D. (2005) Successive chromosome walking by compatible ends ligation inverse PCR. Molecular Biotechnology 30: 95-101

Starks C.M., Back K., Chappell J., Noel J.P. (1997) Structural basis for cyclic terpene biosynthesis by tobacco 5-epi-aristolochene synthase. Science 277: 1815-1820

Steel R. D., Torrie J. H., Dickey D. A. (1997) Principles and procedures of statistics: a biometrical approach (McGraw-Hill, New York, 1997).

Thompson J.D., Gibson T.J., Plewniak F., Jeanmougin F., Higgins D.G. (1997) The CLUSTAL_X windows interface: flexible strategies for multiple sequence alignment aided by quality analysis tools. Nucleic Acids Research 25: 4876-4882

Torney F., Partier A., Says-Lesage V., Nadaud I., Barret P., Beckert M. (2004) Heritable transgene expression pattern imposed onto maize ubiquitin promoter by maize adh-1 matrix attachment regions: tissue and developmental specificity in maize transgenic plants. Plant Cell Reporter 22:931-938

Turner G., Gershenzon J., Nielson E.E., Froehlich J.E., Croteau R. (1999) Limonene synthase, the enzyme responsible for monoterpene biosynthesis in peppermint, is localized to leucoplasts of oil gland secretory cells. Plant Physiology 120: 879-886

Van de Peer Y., De Wachter R. (1994) Treecon for Windows - a Software Package for the Construction and Drawing of Evolutionary Trees for the Microsoft Windows Environment. Computer Applications in the Biosciences 10: 569-570

Vijaybhaskar, V., Subbiah, V., Kaur, J., Vijayakumari, P., Siddiqi, I. (2008) Identification of a root-specific glycosyltransferase from Arabidpsis and characterization of its promoter J Biosci 33:185-193

Wise M.L., Savage T.J., Katahira E., Croteau R. (1998) Monoterpene synthases from common sage (Salvia officinalis) - cDNA isolation, characterization, and functional expression of (+)-sabinene synthase, 1,8-cineole synthase, and (+)-bornyl diphosphate synthase. Journal of Biological Chemistry 273: 14891-14899

Wright, R. J., Witkowski, J. F., Echtenkamp, G. & Georgis, R. (1993) Efficacy and persistence of Steinernema carpocapsae (Rhabditida, Steinernematidae) applied through a center-pivot irrigation system against larval Corn Rootworms (Coleoptera, Chrysomelidae). Journal of Economic Entomology 86: 1348-1354

Yu X.H., Liu C.J. (2006) Development of an analytical method for genome-wide functional identification of plant acyl-coenzyme A-dependent acyltransferases. Analytical Biochemistry 358: 146-148

Zavala-Paramo G, Chavez-Moctezuma MP, Garcia-Pineda E, Yin S, Chappell J, Lozoya-Gloria E (2000) Isolation of an elicitor-stimulated 5-epi-aristolochene synthase gene (gPEAS1) from chili pepper (Capsicum annuum). Physiologia Plantarum 110: 410-418.

## Claims

1. A polynucleotide selected from the group consisting of
(a) polynucleotides comprising a nucleotide sequence encoding a polypeptide having the amino acid sequence shown in SEQ ID NO: 2 or 4;
(b) polynucleotides comprising the nucleotide sequence shown in SEQ ID NO:1 or 3;
(c) polynucleotides comprising a nucleotide sequence encoding a fragment of the polypeptide encoded by a polynucleotide of (a) or (b), wherein said nucleotide sequence encodes a protein having caryophyllene synthase activity;
(d) polynucleotides comprising a nucleotide sequence the complementary strand of which hybridizes to the polynucleotide of any one of (a) to (c), wherein said nucleotide sequence encodes a protein having caryophyllene synthase activity;
(e) polynucleotides encoding a polypeptide having an amino acid sequence which is at least 50% identical to the amino acid sequence of a polypeptide encoded by a polynucleotide of any one of (a) to (c) and having caryophyllene synthase activity; and
(f) polynucleotides comprising a nucleotide sequence that deviates from the nucleotide sequence defined in (d) or (e) by the degeneracy of the genetic code.

2. A polynucleotide having a length of at least 15 nucleotides which specifically hybridizes with a polynucleotide of claim 1 or with the complementary strand thereof.

3. A recombinant nucleic acid molecule comprising the polynucleotide of claim 1 or 2 and expression control sequences operably linked to said polynucleotide.

4. The recombinant nucleic acid molecule of claim 3, wherein said expression control sequences comprise a root-specific promoter.

5. A vector comprising the polynucleotide of claim 1 or 2 or the recombinant nucleic acid molecule of claim 3 or 4.

6. A host cell which is genetically engineered with the polynucleotide of claim 1 or 2, the recombinant nucleic acid molecule of claim 3 or 4 or the vector of claim 5.

7. A method for the production of a polypeptide encoded by a polynucleotide of claim 1 or 2 in which the host cell of claim 6 is cultivated under conditions allowing for the expression of the polypeptide and in which the polypeptide is isolated from the cells and/or the culture medium.

8. A polypeptide encoded by the polynucleotide of claim 1 or 2 or obtainable by the method of claim 7.

9. A binding molecule specifically recognizing the polypeptide of claim 8.

10. A transgenic plant cell which is genetically engineered with the polynucleotide of claim 1 or 2, the recombinant nucleic acid molecule of claim 3 or 4 or the vector of claim 5.

11. A transgenic plant or plant tissue comprising the plant cells of claim 10.

12. The transgenic plant of claim 11 which is a maize plant.

13. Propagation material or harvestable parts of the transgenic plant of claim 11 or 12 comprising the plant cells of claim 10.

14. A method for conferring resistance or increased resistance against a herbivore to a plant comprising the step of providing a transgenic plant in which polypeptide of claim 8 is expressed.

15. Use of the polynucleotide of claim 1 or 2, of the recombinant nucleic acid molecule of claim 3 or 4, of the vector of claim 5, of the host cell of claim 6, of the polypeptide of claim 8, of the binding molecule of claim 9 or of the transgenic plant of claim 11 or 12 for establishing or enhancing resistance against a herbivore in plants.

16. The method of claim 14 or the use of claim 15, wherein said herbivore is a root-damaging insect.

17. The method or the use of claim 16, wherein the plants are maize plants and the root-damaging insect is the corn rootworm.

18. Use of the transgenic plant of claim 11 or 12 or of the propagation material of claim 13 together with entomopathogenic nematodes for growing said plant.

19. The use of said claim 18, wherein said transgenic plant or plant is maize, said propagation material is from a maize plant and said entomopathogenic nematode is Heterorhabditis megidis.


SEQUENCE LISTING

<110> Max-Planck Gesellschaft zur Förderung der Wissenschaften
e.V. et al.

<120> Polynucleotides encoding caryophyllene synthase and uses thereof

<130> P2551 EP S3

<160> 4

<170> PatentIn version 3.4

<210> 1
<211> 1665
<212> DNA
<213> Origanum vulgare

<220>
<221> CDS
<222> (1)..(1662)

<400> 1
atg gaa ttt ccg gca tcg gtt gct tcc ctt tcg gct aac acc gtc ggg 48
Met Glu Phe Pro Ala Ser Val Ala Ser Leu Ser Ala Asn Thr Val Gly
1               5                   10                  15

tcg aac gat gtt ctt cgt cgc tcc atc gct tac cat ccc aac att tgg 96
Ser Asn Asp Val Leu Arg Arg Ser Ile Ala Tyr His Pro Asn Ile Trp
            20                  25                  30

gga gac ttt ttt ctt gcg cat act tca gaa ttt atg gaa atc tcg att 144
Gly Asp Phe Phe Leu Ala His Thr Ser Glu Phe Met Glu Ile Ser Ile
        35                  40                  45

gct gaa aag gaa gag cac gaa cgg cta aaa gag gag atc aag aaa ctg 192
Ala Glu Lys Glu Glu His Glu Arg Leu Lys Glu Glu Ile Lys Lys Leu
    50                  55                  60

ctc gtt caa act gag tat gat tcg ata ctt aaa tta gaa ctc atc gat 240
Leu Val Gln Thr Glu Tyr Asp Ser Ile Leu Lys Leu Glu Leu Ile Asp
65                  70                  75                  80

tca att caa cga ctt gga gtg ggt tac cat ttc gaa aaa gaa att gat 288
Ser Ile Gln Arg Leu Gly Val Gly Tyr His Phe Glu Lys Glu Ile Asp
                85                  90                  95

aga atc tta cga tat gtt cat caa acc tac cca ata tac gac acc gaa 336
Arg Ile Leu Arg Tyr Val His Gln Thr Tyr Pro Ile Tyr Asp Thr Glu
            100                 105                 110

aac aag gat ctt cgt atg ctt gct cta cgc ttt cgc cta ctt cga caa 384
Asn Lys Asp Leu Arg Met Leu Ala Leu Arg Phe Arg Leu Leu Arg Gln
        115                 120                 125

caa ggt ttc cat gtt cct ttc gat gtg ctt agt gaa ttc ata gat gcc 432
Gln Gly Phe His Val Pro Phe Asp Val Leu Ser Glu Phe Ile Asp Ala
    130                 135                 140

gaa gga aat ctc acg gag tca ata gca tac gac att caa ggg ata ttg 480
Glu Gly Asn Leu Thr Glu Ser Ile Ala Tyr Asp Ile Gln Gly Ile Leu
145                 150                 155                 160

agc tta tat gag gca tca aat tat gga gtg ctc gga gag gaa att ctt 528
Ser Leu Tyr Glu Ala Ser Asn Tyr Gly Val Leu Gly Glu Glu Ile Leu
                165                 170                 175

gac aaa gca tta gat tcg tgc tcg tct cgt ctt gaa tcg tta atc acg 576
Asp Lys Ala Leu Asp Ser Cys Ser Ser Arg Leu Glu Ser Leu Ile Thr
            180                 185                 190

gac acg gat gat gat cgt ctt tca aga caa gtt aag gaa gct cta aag 624
Asp Thr Asp Asp Asp Arg Leu Ser Arg Gln Val Lys Glu Ala Leu Lys
        195                 200                 205

atc cca att agc aag act cta aca agg ttg gga gca aga aag ttc att 672
Ile Pro Ile Ser Lys Thr Leu Thr Arg Leu Gly Ala Arg Lys Phe Ile
    210                 215                 220

tct atg tac aaa gaa gac gac tct cac aat gaa aaa cta ctc aag ttt 720
Ser Met Tyr Lys Glu Asp Asp Ser His Asn Glu Lys Leu Leu Lys Phe
225                 230                 235                 240

gcc atg ttg gac ttt aac atg gta caa agg cta cat caa aat gag ctt 768
Ala Met Leu Asp Phe Asn Met Val Gln Arg Leu His Gln Asn Glu Leu
                245                 250                 255

agc cat ctt aca aga tgg tgg aag gaa tta gac ttt gca aac aag ctg 816
Ser His Leu Thr Arg Trp Trp Lys Glu Leu Asp Phe Ala Asn Lys Leu
            260                 265                 270

cct ttt gct agg gat aga ctt gtg gag tgc tac ttc tgg att atg gga 864
Pro Phe Ala Arg Asp Arg Leu Val Glu Cys Tyr Phe Trp Ile Met Gly
        275                 280                 285

gtt tat tat gag ccg cgt cac gaa att gcc cga aaa ata cta acc aaa 912
Val Tyr Tyr Glu Pro Arg His Glu Ile Ala Arg Lys Ile Leu Thr Lys
    290                 295                 300

gtc att tac atg gct tct gtc ctc gat gac att tac gac gtc tat gga 960
Val Ile Tyr Met Ala Ser Val Leu Asp Asp Ile Tyr Asp Val Tyr Gly
305                 310                 315                 320

aca tta gac gag ctc aca cta ttc act agc ttt gtt cga agg tgg gac 1008
Thr Leu Asp Glu Leu Thr Leu Phe Thr Ser Phe Val Arg Arg Trp Asp
                325                 330                 335

att agt ggc att gat gaa ttg cca aca tac atg aga ata tat ttg aga 1056
Ile Ser Gly Ile Asp Glu Leu Pro Thr Tyr Met Arg Ile Tyr Leu Arg
            340                 345                 350

gcc ctt ttc gat gtg tat gtt gaa atg gaa gaa gaa atg gga aaa ata 1104
Ala Leu Phe Asp Val Tyr Val Glu Met Glu Glu Glu Met Gly Lys Ile
        355                 360                 365

ggc aaa tca tac gca atc gaa tat gca aaa gaa gag atg aaa aga ttg 1152
Gly Lys Ser Tyr Ala Ile Glu Tyr Ala Lys Glu Glu Met Lys Arg Leu
    370                 375                 380

gca gag gtg tac ttt caa gag gca caa tgg ttc ttt agc aag tac aaa 1200
Ala Glu Val Tyr Phe Gln Glu Ala Gln Trp Phe Phe Ser Lys Tyr Lys
385                 390                 395                 400

cct aca atg caa gag tat atg aag gtg gct ctc tta tcg tct ggt tat 1248
Pro Thr Met Gln Glu Tyr Met Lys Val Ala Leu Leu Ser Ser Gly Tyr
                405                 410                 415

atg atg atg aca atc aat tca tta gct gtt atc aaa gat cca att acc 1296
Met Met Met Thr Ile Asn Ser Leu Ala Val Ile Lys Asp Pro Ile Thr
            420                 425                 430

aag aaa gag ttt gat tgg gtt gtg agc gaa cca cca att cta aag tca 1344
Lys Lys Glu Phe Asp Trp Val Val Ser Glu Pro Pro Ile Leu Lys Ser
        435                 440                 445

tca tca atc att aca aga tta atg gac gac ctt gcg gga tat ggg tct 1392
Ser Ser Ile Ile Thr Arg Leu Met Asp Asp Leu Ala Gly Tyr Gly Ser
    450                 455                 460

gaa gag aaa cac tca gca gtg cat ctg tac atg aat gaa aag ggc gta 1440
Glu Glu Lys His Ser Ala Val His Leu Tyr Met Asn Glu Lys Gly Val
465                 470                 475                 480

tcg gag gaa gaa gcc ttt caa gaa ctc cgc aaa caa gtg aaa aac tca 1488
Ser Glu Glu Glu Ala Phe Gln Glu Leu Arg Lys Gln Val Lys Asn Ser
                485                 490                 495

tgg aag aat ata aac aag gaa tgt ctg aag cta cga cca gcc tct gtt 1536
Trp Lys Asn Ile Asn Lys Glu Cys Leu Lys Leu Arg Pro Ala Ser Val
            500                 505                 510

cca atc ctt acg acc gtc gtc aat ttc aca cgt gtt ata atc gtg ttg 1584
Pro Ile Leu Thr Thr Val Val Asn Phe Thr Arg Val Ile Ile Val Leu
        515                 520                 525

tat aca gat gaa gat gca tat ggt aat tct aaa act aag act aaa gat 1632
Tyr Thr Asp Glu Asp Ala Tyr Gly Asn Ser Lys Thr Lys Thr Lys Asp
    530                 535                 540

atg atc aaa tcc ata ctc gtt gat ccc gta taa 1665
Met Ile Lys Ser Ile Leu Val Asp Pro Val
545                 550

<210> 2
<211> 554
<212> PRT
<213> Origanum vulgare

<400> 2

Met Glu Phe Pro Ala Ser Val Ala Ser Leu Ser Ala Asn Thr Val Gly
1               5                   10                  15

Ser Asn Asp Val Leu Arg Arg Ser Ile Ala Tyr His Pro Asn Ile Trp
            20                  25                  30

Gly Asp Phe Phe Leu Ala His Thr Ser Glu Phe Met Glu Ile Ser Ile
        35                  40                  45

Ala Glu Lys Glu Glu His Glu Arg Leu Lys Glu Glu Ile Lys Lys Leu
    50                  55                  60

Leu Val Gln Thr Glu Tyr Asp Ser Ile Leu Lys Leu Glu Leu Ile Asp
65                  70                  75                  80

Ser Ile Gln Arg Leu Gly Val Gly Tyr His Phe Glu Lys Glu Ile Asp
                85                  90                  95

Arg Ile Leu Arg Tyr Val His Gln Thr Tyr Pro Ile Tyr Asp Thr Glu
            100                 105                 110

Asn Lys Asp Leu Arg Met Leu Ala Leu Arg Phe Arg Leu Leu Arg Gln
        115                 120                 125

Gln Gly Phe His Val Pro Phe Asp Val Leu Ser Glu Phe Ile Asp Ala
    130                 135                 140

Glu Gly Asn Leu Thr Glu Ser Ile Ala Tyr Asp Ile Gln Gly Ile Leu
145                 150                 155                 160

Ser Leu Tyr Glu Ala Ser Asn Tyr Gly Val Leu Gly Glu Glu Ile Leu
                165                 170                 175

Asp Lys Ala Leu Asp Ser Cys Ser Ser Arg Leu Glu Ser Leu Ile Thr
            180                 185                 190

Asp Thr Asp Asp Asp Arg Leu Ser Arg Gln Val Lys Glu Ala Leu Lys
        195                 200                 205

Ile Pro Ile Ser Lys Thr Leu Thr Arg Leu Gly Ala Arg Lys Phe Ile
    210                 215                 220

Ser Met Tyr Lys Glu Asp Asp Ser His Asn Glu Lys Leu Leu Lys Phe
225                 230                 235                 240

Ala Met Leu Asp Phe Asn Met Val Gln Arg Leu His Gln Asn Glu Leu
                245                 250                 255

Ser His Leu Thr Arg Trp Trp Lys Glu Leu Asp Phe Ala Asn Lys Leu
            260                 265                 270

Pro Phe Ala Arg Asp Arg Leu Val Glu Cys Tyr Phe Trp Ile Met Gly
        275                 280                 285

Val Tyr Tyr Glu Pro Arg His Glu Ile Ala Arg Lys Ile Leu Thr Lys
    290                 295                 300

Val Ile Tyr Met Ala Ser Val Leu Asp Asp Ile Tyr Asp Val Tyr Gly
305                 310                 315                 320

Thr Leu Asp Glu Leu Thr Leu Phe Thr Ser Phe Val Arg Arg Trp Asp
                325                 330                 335

Ile Ser Gly Ile Asp Glu Leu Pro Thr Tyr Met Arg Ile Tyr Leu Arg
            340                 345                 350

Ala Leu Phe Asp Val Tyr Val Glu Met Glu Glu Glu Met Gly Lys Ile
        355                 360                 365

Gly Lys Ser Tyr Ala Ile Glu Tyr Ala Lys Glu Glu Met Lys Arg Leu
    370                 375                 380

Ala Glu Val Tyr Phe Gln Glu Ala Gln Trp Phe Phe Ser Lys Tyr Lys
385                 390                 395                 400

Pro Thr Met Gln Glu Tyr Met Lys Val Ala Leu Leu Ser Ser Gly Tyr
                405                 410                 415

Met Met Met Thr Ile Asn Ser Leu Ala Val Ile Lys Asp Pro Ile Thr
            420                 425                 430

Lys Lys Glu Phe Asp Trp Val Val Ser Glu Pro Pro Ile Leu Lys Ser
        435                 440                 445

Ser Ser Ile Ile Thr Arg Leu Met Asp Asp Leu Ala Gly Tyr Gly Ser
    450                 455                 460

Glu Glu Lys His Ser Ala Val His Leu Tyr Met Asn Glu Lys Gly Val
465                 470                 475                 480

Ser Glu Glu Glu Ala Phe Gln Glu Leu Arg Lys Gln Val Lys Asn Ser
                485                 490                 495

Trp Lys Asn Ile Asn Lys Glu Cys Leu Lys Leu Arg Pro Ala Ser Val
            500                 505                 510

Pro Ile Leu Thr Thr Val Val Asn Phe Thr Arg Val Ile Ile Val Leu
        515                 520                 525

Tyr Thr Asp Glu Asp Ala Tyr Gly Asn Ser Lys Thr Lys Thr Lys Asp
    530                 535                 540

Met Ile Lys Ser Ile Leu Val Asp Pro Val
545                 550

<210> 3
<211> 1665
<212> DNA
<213> Origanum vulgare

<220>
<221> CDS
<222> (1)..(1662)

<400> 3
atg gaa ttt ccg gca tcg gtt gct tcc ctt tcg gct aac acc gtc ggg 48
Met Glu Phe Pro Ala Ser Val Ala Ser Leu Ser Ala Asn Thr Val Gly
1               5                   10                  15

tcg aac gat gtt ctt cgt cgc tcc gtc gct tac cat ccc aac att tgg 96
Ser Asn Asp Val Leu Arg Arg Ser Val Ala Tyr His Pro Asn Ile Trp
            20                  25                  30

gga gac ttt ttt ctt gcg cat act tca gaa ttt atg gaa atc tcg att 144
Gly Asp Phe Phe Leu Ala His Thr Ser Glu Phe Met Glu Ile Ser Ile
        35                  40                  45

gct gaa aag gaa gag cac gaa tgg cta aaa gag gag atc aag aaa ttg 192
Ala Glu Lys Glu Glu His Glu Trp Leu Lys Glu Glu Ile Lys Lys Leu
    50                  55                  60

ctc gtt caa act gag tat gat tcg ata ctt aaa tta gaa ctc atc gat 240
Leu Val Gln Thr Glu Tyr Asp Ser Ile Leu Lys Leu Glu Leu Ile Asp
65                  70                  75                  80

tca atc caa cga ctt gga gtg agt tac cat ttc gaa aaa gaa att gat 288
Ser Ile Gln Arg Leu Gly Val Ser Tyr His Phe Glu Lys Glu Ile Asp
                85                  90                  95

aga atc tta cga tat gtt cat caa acc tac cca ata tac gag acc gaa 336
Arg Ile Leu Arg Tyr Val His Gln Thr Tyr Pro Ile Tyr Glu Thr Glu
            100                 105                 110

aac aag gat ctt cgt atg ctt gct cta cgc ttt cgc cta ctt cga caa 384
Asn Lys Asp Leu Arg Met Leu Ala Leu Arg Phe Arg Leu Leu Arg Gln
        115                 120                 125

cat ggt ttc cat gtt cct tgc gat gtg ttt agt gaa ttc ata gat gcc 432
His Gly Phe His Val Pro Cys Asp Val Phe Ser Glu Phe Ile Asp Ala
    130                 135                 140

gaa gga aat ctc atg gag tca ata gca tac gac att caa ggg ata ttg 480
Glu Gly Asn Leu Met Glu Ser Ile Ala Tyr Asp Ile Gln Gly Ile Leu
145                 150                 155                 160

agc tta tat gag gca tca aat tat gga gtg ctc gga gag gaa att ctt 528
Ser Leu Tyr Glu Ala Ser Asn Tyr Gly Val Leu Gly Glu Glu Ile Leu
                165                 170                 175

gac aaa gca tta gat tcg tgc tcg tct cat ctt gaa tcg tta atc acg 576
Asp Lys Ala Leu Asp Ser Cys Ser Ser His Leu Glu Ser Leu Ile Thr
            180                 185                 190

gac acg aat gat gat cgt ctt tca aga caa gtt aag gaa gct cta aag 624
Asp Thr Asn Asp Asp Arg Leu Ser Arg Gln Val Lys Glu Ala Leu Lys
        195                 200                 205

atc cca att agc aag act cta aca agg ttg gga gca aga aag ttc att 672
Ile Pro Ile Ser Lys Thr Leu Thr Arg Leu Gly Ala Arg Lys Phe Ile
    210                 215                 220

tct atg tac aaa gaa gac gac tct cac aat gaa aaa cta ctc aag ttt 720
Ser Met Tyr Lys Glu Asp Asp Ser His Asn Glu Lys Leu Leu Lys Phe
225                 230                 235                 240

gcc atg ttg gac ttt aac atg gta caa agg cta cat caa aat gag ctt 768
Ala Met Leu Asp Phe Asn Met Val Gln Arg Leu His Gln Asn Glu Leu
                245                 250                 255

agc cat ctt aca agt tgg tgg aag gaa tta gac ttt gca aac aag ctg 816
Ser His Leu Thr Ser Trp Trp Lys Glu Leu Asp Phe Ala Asn Lys Leu
            260                 265                 270

cct ttt gct agg gat aga ctt gtg gag tgc tac ttc tgg att atg gga 864
Pro Phe Ala Arg Asp Arg Leu Val Glu Cys Tyr Phe Trp Ile Met Gly
        275                 280                 285

gtt tat ttt gag ccg cgt cac gaa att gcc cga aaa ata cta acc aaa 912
Val Tyr Phe Glu Pro Arg His Glu Ile Ala Arg Lys Ile Leu Thr Lys
    290                 295                 300

gtc att tac atg gct tct gtc ctt gat gac act tac gac gtc tat gga 960
Val Ile Tyr Met Ala Ser Val Leu Asp Asp Thr Tyr Asp Val Tyr Gly
305                 310                 315                 320

aca tta gac gag ctt ata cta ttc act agc gtt gtt cga agg tgg gac 1008
Thr Leu Asp Glu Leu Ile Leu Phe Thr Ser Val Val Arg Arg Trp Asp
                325                 330                 335

att agt ggc att gat gaa ttg cca aca tac atg aga ata tac ttg aga 1056
Ile Ser Gly Ile Asp Glu Leu Pro Thr Tyr Met Arg Ile Tyr Leu Arg
            340                 345                 350

gcc ctt ttc gat gtg tat gtt gaa atg gaa gaa gaa atg gga aaa ata 1104
Ala Leu Phe Asp Val Tyr Val Glu Met Glu Glu Glu Met Gly Lys Ile
        355                 360                 365

ggc aaa tca tac gca gtc gaa tat gca aaa gaa gag atg aaa aga ttg 1152
Gly Lys Ser Tyr Ala Val Glu Tyr Ala Lys Glu Glu Met Lys Arg Leu
    370                 375                 380

gca gag gtg tac ttt caa gag gca caa tgg ttc ttt agc aag tac aaa 1200
Ala Glu Val Tyr Phe Gln Glu Ala Gln Trp Phe Phe Ser Lys Tyr Lys
385                 390                 395                 400

cct aca atg caa gag tat atg aag gtg gct ctc tta tcg tct ggt tat 1248
Pro Thr Met Gln Glu Tyr Met Lys Val Ala Leu Leu Ser Ser Gly Tyr
                405                 410                 415

atg atg atg aca atc aat tca tta gct gtt atc gaa gat cca att acc 1296
Met Met Met Thr Ile Asn Ser Leu Ala Val Ile Glu Asp Pro Ile Thr
            420                 425                 430

aag aaa gag ttt gat tgg gtt gtg agc gaa cca cca att cta aag tca 1344
Lys Lys Glu Phe Asp Trp Val Val Ser Glu Pro Pro Ile Leu Lys Ser
        435                 440                 445

tca tca atc att aca aga tta atg gac gac ctt gcg gga tat ggg tct 1392
Ser Ser Ile Ile Thr Arg Leu Met Asp Asp Leu Ala Gly Tyr Gly Ser
    450                 455                 460

gaa gat aaa tac tca gca gta cat ctg tac atg aac gaa aag ggc gta 1440
Glu Asp Lys Tyr Ser Ala Val His Leu Tyr Met Asn Glu Lys Gly Val
465                 470                 475                 480

tcg gag gaa gaa gcc ttt caa gaa ctc cgc aaa caa gtg aaa aac tca 1488
Ser Glu Glu Glu Ala Phe Gln Glu Leu Arg Lys Gln Val Lys Asn Ser
                485                 490                 495

tgg aag aat aga aac aag gaa tgt ctg gag cca cga tca gcc tct gtt 1536
Trp Lys Asn Arg Asn Lys Glu Cys Leu Glu Pro Arg Ser Ala Ser Val
            500                 505                 510

cca att ctt acg acc gtc gtc aat ttc aca cgt gtc gta gtc gtg ttg 1584
Pro Ile Leu Thr Thr Val Val Asn Phe Thr Arg Val Val Val Val Leu
        515                 520                 525

tat aca gat gaa gat gca tat ggc aat tcc aaa aat aag act aaa gat 1632
Tyr Thr Asp Glu Asp Ala Tyr Gly Asn Ser Lys Asn Lys Thr Lys Asp
    530                 535                 540

atg atc aaa tcc ata ctc gtt gat ccc gta taa 1665
Met Ile Lys Ser Ile Leu Val Asp Pro Val
545                 550

<210> 4
<211> 554
<212> PRT
<213> Origanum vulgare

<400> 4

Met Glu Phe Pro Ala Ser Val Ala Ser Leu Ser Ala Asn Thr Val Gly
1               5                   10                  15

Ser Asn Asp Val Leu Arg Arg Ser Val Ala Tyr His Pro Asn Ile Trp
            20                  25                  30

Gly Asp Phe Phe Leu Ala His Thr Ser Glu Phe Met Glu Ile Ser Ile
        35                  40                  45

Ala Glu Lys Glu Glu His Glu Trp Leu Lys Glu Glu Ile Lys Lys Leu
    50                  55                  60

Leu Val Gln Thr Glu Tyr Asp Ser Ile Leu Lys Leu Glu Leu Ile Asp
65                  70                  75                  80

Ser Ile Gln Arg Leu Gly Val Ser Tyr His Phe Glu Lys Glu Ile Asp
                85                  90                  95

Arg Ile Leu Arg Tyr Val His Gln Thr Tyr Pro Ile Tyr Glu Thr Glu
            100                 105                 110

Asn Lys Asp Leu Arg Met Leu Ala Leu Arg Phe Arg Leu Leu Arg Gln
        115                 120                 125

His Gly Phe His Val Pro Cys Asp Val Phe Ser Glu Phe Ile Asp Ala
    130                 135                 140

Glu Gly Asn Leu Met Glu Ser Ile Ala Tyr Asp Ile Gln Gly Ile Leu
145                 150                 155                 160

Ser Leu Tyr Glu Ala Ser Asn Tyr Gly Val Leu Gly Glu Glu Ile Leu
                165                 170                 175

Asp Lys Ala Leu Asp Ser Cys Ser Ser His Leu Glu Ser Leu Ile Thr
            180                 185                 190

Asp Thr Asn Asp Asp Arg Leu Ser Arg Gln Val Lys Glu Ala Leu Lys
        195                 200                 205

Ile Pro Ile Ser Lys Thr Leu Thr Arg Leu Gly Ala Arg Lys Phe Ile
    210                 215                 220

Ser Met Tyr Lys Glu Asp Asp Ser His Asn Glu Lys Leu Leu Lys Phe
225                 230                 235                 240

Ala Met Leu Asp Phe Asn Met Val Gln Arg Leu His Gln Asn Glu Leu
                245                 250                 255

Ser His Leu Thr Ser Trp Trp Lys Glu Leu Asp Phe Ala Asn Lys Leu
            260                 265                 270

Pro Phe Ala Arg Asp Arg Leu Val Glu Cys Tyr Phe Trp Ile Met Gly
        275                 280                 285

Val Tyr Phe Glu Pro Arg His Glu Ile Ala Arg Lys Ile Leu Thr Lys
    290                 295                 300

Val Ile Tyr Met Ala Ser Val Leu Asp Asp Thr Tyr Asp Val Tyr Gly
305                 310                 315                 320

Thr Leu Asp Glu Leu Ile Leu Phe Thr Ser Val Val Arg Arg Trp Asp
                325                 330                 335

Ile Ser Gly Ile Asp Glu Leu Pro Thr Tyr Met Arg Ile Tyr Leu Arg
            340                 345                 350

Ala Leu Phe Asp Val Tyr Val Glu Met Glu Glu Glu Met Gly Lys Ile
        355                 360                 365

Gly Lys Ser Tyr Ala Val Glu Tyr Ala Lys Glu Glu Met Lys Arg Leu
    370                 375                 380

Ala Glu Val Tyr Phe Gln Glu Ala Gln Trp Phe Phe Ser Lys Tyr Lys
385                 390                 395                 400

Pro Thr Met Gln Glu Tyr Met Lys Val Ala Leu Leu Ser Ser Gly Tyr
                405                 410                 415

Met Met Met Thr Ile Asn Ser Leu Ala Val Ile Glu Asp Pro Ile Thr
            420                 425                 430

Lys Lys Glu Phe Asp Trp Val Val Ser Glu Pro Pro Ile Leu Lys Ser
        435                 440                 445

Ser Ser Ile Ile Thr Arg Leu Met Asp Asp Leu Ala Gly Tyr Gly Ser
    450                 455                 460

Glu Asp Lys Tyr Ser Ala Val His Leu Tyr Met Asn Glu Lys Gly Val
465                 470                 475                 480

Ser Glu Glu Glu Ala Phe Gln Glu Leu Arg Lys Gln Val Lys Asn Ser
                485                 490                 495

Trp Lys Asn Arg Asn Lys Glu Cys Leu Glu Pro Arg Ser Ala Ser Val
            500                 505                 510

Pro Ile Leu Thr Thr Val Val Asn Phe Thr Arg Val Val Val Val Leu
        515                 520                 525

Tyr Thr Asp Glu Asp Ala Tyr Gly Asn Ser Lys Asn Lys Thr Lys Asp
    530                 535                 540

Met Ile Lys Ser Ile Leu Val Asp Pro Val
545                 550